# EUROPEAN PATENT SPECIFICATION

(11) **EP 1 188 821 B1**
(45) Date of publication and mention of the grant of the patent: **15.08.2007**
(21) Application number: 01917781.5
(22) Date of filing: 02.04.2001
(51) Int. Cl.: C12M 3/00, C12N 5/06, C12N 11/16

(54) **CARRIER FOR ANIMAL CELL CULTURE COMPRISING ORGAN TISSUE SLICE AND ANIMAL CELL CULTURE METHOD AND TRANSPLANTATION METHOD WITH THE USE OF THE CARRIER**
TRäGER FüR TIERZELLKULTUR, WELCHER EINE ORGANGEWEBESCHEIBE BEINHALTET UND TIERZELLKULTUR-VERFAHREN UND TRANSPLANTATIONSVERFAHREN UNTER VERWENDUNG EINES SOLCHEN TRäGERS
PORTEUR POUR CULTURE CELLULAIRE ANIMALE COMPRENANT UNE COUPE BIOLOGIQUE D'UN ORGANE, PROCEDE DE CULTURE CELLULAIRE ANIMALE ET PROCEDE DE TRANSPLANTATION REPOSANT SUR L'UTILISATION DE CE PORTEUR

(30) Priority: 31.03.2000 JP 2000098401; 30.03.2001 JP 2001101961
(43) Date of publication of application: 20.03.2002
(73) Proprietor: Incorporated Administrative Agency National Agriculture and Bio-oriented Research Organization, Ibaraki 305-0856 (JP); Takezawa, Toshiaki, Machida-shi, Tokyo 194-0211 (JP)
(72) Inventor: TAKEZAWA, Toshiaki, Machida-shi, Tokyo 194-0211 (JP); HASHIZUME, Kazuyoshi, Katsushika-ku, Tokyo 124-0006 (JP); IMAI, Kei, Tsukuba-shi, Ibaraki 305-0044 (JP); TAKAHASHI, Toru, Tsukuba-shi, Ibaraki 305-0035 (JP)
(74) Representative: Webber, Philip Michael
(86) International application number: PCT/JP2001/002859
(87) International publication number: WO 2001/072953

(56) References cited:
- WO-A-90/02796
- WO-A1-90/02796
- JP-A- 9 154 567
- BADER A ET AL: "Tissue engineering of heart valves--human endothelial cell seeding of detergent acellularized porcine valves." EUROPEAN JOURNAL OF CARDIO-THORACIC SURGERY: OFFICIAL JOURNAL OF THE EUROPEAN ASSOCIATION FOR CARDIO-THORACIC SURGERY. NETHERLANDS SEP 1998, vol. 14, no. 3, September 1998 (1998-09), pages 279-284, XP002209841 ISSN: 1010-7940
- MORI T ET AL: "PERENCHYMAL CELLS PROLIFERATE AND DIFFERENTIATE IN A ORGANOTYPIC SLICE CULTURE OF THE NEONATAL LIVER" ANATOMY AND EMBRYOLOGY, SPRINGER INTERNATIONAL, BERLIN, DE, vol. 199, 1999, pages 319-327, XP002941732 ISSN: 0340-2061
- BARBER R P ET AL: "Autonomic motor neuron migration and expression of nicotinamide adenine dinucleotide phosphate reduced diaphorase are dependent upon peripheral target." THE JOURNAL OF COMPARATIVE NEUROLOGY. UNITED STATES 7 SEP 1998, vol. 398, no. 4, 7 September 1998 (1998-09-07), pages 568-574, XP002209738 ISSN: 0021-9967
- WETTS R ET AL: "Peripheral and central target requirements for survival of embryonic rat dorsal root ganglion neurons in slice cultures." THE JOURNAL OF NEUROSCIENCE: THE OFFICIAL JOURNAL OF THE SOCIETY FOR NEUROSCIENCE. UNITED STATES 1 SEP 1998, vol. 18, no. 17, 1 September 1998 (1998-09-01), pages 6905-6913, XP002209739 ISSN: 0270-6474
- TAKAHIRO MORI ET AL.: 'Parenchymal cells proliferate and differentiate in an organotypic slice culture of the neonatal liver' ANATOMY AND EMBRYOLOGY vol. 199, no. 4, 1999, pages 319 - 327, XP002941732

## Description

### TECHNICAL FIELD

The invention of this application relates to a cell culture system comprising a tissue section-containing carrier for culture of animal cells, and to a method of using the cell culture system for culture of animal cells. More precisely, the invention of this application relates to a cell culture system comprising a tissue section-containing carrier for culture of animal cells, which provides the site and time-dependent environmental information on the tissue for the animal cells being cultured with it; and to a method of culturing animal cells on the culture carrier. The invention realizes culture of various cells on various tissue-derived carriers. Taking advantage of the carrier, the invention enables culture and maintenance of functional cells, evaluation of cell behavior and function and biological tissue reconstruction, and is therefore useful, for example, for evaluation of gene function, development of lab-grown organs and cell transplantation.

### BACKGROUND ART

The application of animal cells cultured in a specifically planned environment is now greatly expanding to the studies for developing and producing physiologically-active agents such as medicines, and to those for producing and transplanting lab -grown organs, and its contribution to the present-day society is remarkable. Heretofore, various carriers for culture of animal cells have been developed for making animal-derived functional cells exhibit the intended potency. This is because specifically planning the materials and the forms of carriers for cell culture makes it possible to control the cell behavior including cell adhesion, spread, migration, invasion, proliferation, differentiation, polarization and self-assembly, and to control the interactive mechanism between cells and culture media.

The conventional materials for carriers for cell culture are classified into natural resource-derived materials, artificial materials, and hybrid materials composed of such natural resource-derived materials and artificial materials. Various forms of the materials are known, including, for example, adsorbents to supports, layers to coat supports, as well as films, membranes, plates, dishes, flasks, hollow fibers, threads and/or their woven matters, gels, beads, etc. For the natural resource-derived materials for cell culture carriers, for example, known are extracellular matrix constituent components extracted from animal tissues, such as collagen, fibronectin, laminin, glycosaminoglycan, proteoglycan, and matrigel a reconstituted (basement membrane prepared from EHS tumor; Kleinman, H. K., et al; Basement membrane complexes with biological activity, Biochem., 25, 312, 1986) ; acellular dermal matrix prepared from animal tissues (Livesey, S. A., et al.; Transplanted acellular allograft dermal matrix, Transplant, 60, 1.1995) ; *Mytilidae*-derived adhesive protein ; and silk and cotton. The artificial materials known for cell culture carriers are non-biodegradable synthetic polymers such as nylon; biodegradable synthetic polymers such as polyglycolic acid; and ceramics.

Regarding the primary culture of animal cells, in general, it is known that, when they are cultured in a plastic dish serving as a culture carrier, they lose the tissue-specific function which they have expressed in vivo; but when cultured by the use of a culture carrier that contains an extracellular matrix constituent component, they may keep the function; and especially when epithelial cells are cultured by the use of amatrigel serving as a culture carrier, they are differentiated and their tissue-specific function is thereby improved. On the other hand, it is also known that, when epidermal karatinocytes are cultured by the use of an acellular dermal matrix serving as a culture carrier, they are differentiated to reconstruct epidermis on the acellular dermal matrix. The knowledge suggests that, for inducing cultured cells to have the intended, tissue-specific function or three-dimensional microstructural morphology, it is important to reflect the site information which the cells naturally have in vivo on the culture carrier as accurately as possible. In animal tissues, the cells time-dependently change from their undifferentiated condition to the terminally differentiated condition, and the extracellular matrix also time-dependently varies in accordance with the differentiated condition of the cells. Therefore, the extracellular matrix environment in which each cell is localized is also governed by the time-dependent change. In other words, the environment that surrounds the cells in living tissues has not only the site-dependent information but also the time-dependent information.

However, no one has heretofore developed a culture carrier that reflects the environment of a living tissue composed of cells in various differentiation conditions, or that is, a culture carrier capable of accurately reflecting the site and time-dependent information on such a living tissue.

Accordingly, it has heretofore been impossible to realize a culture system capable of imparting an extracellular environment well similar to the *in-vivo* condition to desired animal cells.

The invention of this application has been made in consideration of the current situation in the art as above, and its object is to provide a carrier for culture of animal cells, with which the culture environment is more similar to that in living tissue, or that is, to provide such a carrier for culture of animal cells which involves the intended, site and time-dependent information on living tissue. In addition, another object of the invention of this application is to culture animal cells on the tissue-derived culture carrier to thereby make it possible to analyze the properties and the characteristics of the cultured cells on the basis of the interaction between the cells and the site and time-dependent information on the tissue of the carrier, to analyze the function of genes on the basis of the interaction therebetween, to reconstruct a novel engineered tissue by utilizing the carrier tissue as a template, and to transplant the reconstructed tissue.

### DISCLOSURE OF THE INVENTION

In order to provide animals cells being cultured in a culture system with a site and time-dependent information on a living tissue, we, the inventors of the invention of this application have found a method of using tissue sections, which are generally prepared for microscopic observation in the field of histology and pathology, for a carrier for the cell culture, directly or after having been processed for acellularization. Accordingly, the first aspect of the invention of this application is to provide a cell culture system comprising a cell culture vessel into which has been fitted a cell culture carrier which contains a tissue section which has been equilibrated with a culture medium, wherein
(i) the tissue section has previously been frozen and the tissue section has a thickness of from 1 to 50 µm,
(ii) the tissue section has previously been embedded in paraffin and the tissue section has a thickness of from 0.5 to 50 µm, or
(iii) the tissue section has previously been embedded in resin and the tissue section has a thickness of from 0.5 to 50 µm, and wherein animal cells have been seeded on the carrier to start the culture thereof, and wherein the said animal cells are animal cell suspensions, tissue explants, non-human blastocysts, or three-dimensionally reconstructed multicellular aggregates.

The second aspect thereof is to provide the cell culture system in which the tissue section is stuck to a support or stuck thereto while stretched; the third aspect thereof is to provide the cell culture system in which the support is at least one selected from glass, plastics, rubber, metal, natural or synthetic thread and/or its woven materials, and biodegradable materials; and the fourth aspect thereof is to provide the cell culture system in which the support is previously processed for promoting the section adhesion thereto or for promoting the section adhesion thereto under stretch.

The fifth aspect of the invention of this application is to provide the cell culture system for which the tissue is prepared from a fresh tissue or a tissue previously fixed by the use of a fixing reagent; the sixth aspect thereof is to provide the cell culture system for which the tissue is processed for acellularization before or after it has been sectioned; the seventh aspect thereof is to provide the cell culture system in which the tissue section is treated with an antibody or a nucleic acid probe capable of binding a physiologically-active agent to thereby introduce an exogenous physiologically-active agent into a specific site of the section, wherein the physiologically active agent is a protein, glycoprotein, lipoprotein, peptide, saccharide, lipid, liposaccharide, nucleic acid or a low molecular weight compound; the eighth aspect thereof is to provide the cell culture system in which the tissue section is biologically treated with enzyme or chemically treated with acid, alkali, surfactant to thereby change or modify the constituent components and/or the microstructure of the tissue section; the ninth aspect thereof is to provide the cell culture system for which the tissue to be sectioned is previously frozen, or embedded by freezing, or embedded in paraffin or resin; the tenth aspect thereof is to provide the cell culture system for which the tissue is derived from animals; the eleventh aspect thereof is to provide the cell culture system for which the tissue is derived from mammals; the twelfth aspect thereof is to provide the cell culture system for which the tissue is derived from a part or all of an unborn non-human animal living in its mother during the developmental stage; the thirteenth aspect thereof is to provide the cell culture system for which the tissue is derived from a part or all of a born animal.

The fourteenth aspect of the invention of this application is to provide a method of cell culture, which comprises fitting the cell culture carrier in a culture vessel, and culturing animal cells therein, in which animal cell suspensions, tissue explants, non-human blastocysts, or three-dimensionally reconstructed multicellular aggregates are seeded on the carrier to start the culture of the animal cells thereon; the fifteenth aspect thereof is to provide the cell culture method in which the animal cells seeded on the carrier adhere, proliferate, and subsequently peel the section or the section-derived tissue with the proliferated cells from the support of any of the second to fourth aspects of the invention; the sixteenth aspect thereof is to provide the cell culture method in which the section or the section-derived tissue with the proliferated cells is, after peeled from the support, further cultured to thereby form three-dimensional multicellular aggregates that have involved the section or the section-derived tissue therein; the seventeenth aspect thereof is to provide the cell culture method in which the animal cells to be cultured are of one or more types selected from primary culture cells, cells of established cell lines, non-human blastocysts and/or those cells with an exogenous gene introduced thereinto; the eighteenth aspect thereof is to provide the cell culture method in which the animal cells to be cultured are derived from any of undifferentiated stem cells, cells under differentiation, terminally differentiated cells and/or dedifferentiated cells, wherein the cells are not human embryonic cells, and such a method in which the undifferentiated stem cells are especially embryonic stem cells; and the nineteenth aspect thereof is to provide the cell culture method of any of the fourteenth to eighteenth aspects, in which the culture medium for culturing the animal cells therein is a serum-containing culture medium or a serum-free culture medium not containing serum.

### BRIEF DESCRIPTION OF THE DRAWINGS

Fig. 1 is a substantial photograph of four slideglasses put in a culture tray, in which a 5 µm-thick, frozen bovine placenta section was stuck to each slideglass while stretched.
Fig. 2 is an optical microscopic photograph of a cell culture carrier of a 5 µm-thick, frozen bovine placenta section stained with hematoxylin-eosin. (The bar on the photograph corresponds to 200 µm.)
Fig. 3 is a substantial photograph of a 35-mmφ hydrophilic culture dish of polystyrene with a 5 µm-thick, paraffin-embedding-derived section of a 13-day estrous nulliparous bovine uterus stuck thereto under stretch.
Fig. 4 is a substantial photograph of a 35-mmφ hydrophobic culture dish of polystyrene with a 5 µm-thick, paraffin-embedding-derived section of a 13-day estrous nulliparous bovine uterus stuck thereto under stretch.
Fig. 5 is an optical microscopic photograph of a cell culture carrier of a 5 µm-thick, frozen bovine placenta section acellularized with 0.01 % SDS and stained with hematoxylin-eosin. (The bar on the photograph corresponds to 200 µm.)
Fig. 6 is an optical microscopic photograph of a cell culture carrier of a 5 µm-thick, frozen bovine placenta section acellularized with 0.1 % SDS and stained with hematoxylin-eosin. (The bar on the photograph corresponds to 200 µm.)
Fig. 7 is an optical microscopic photograph of a cell culture carrier of a 5 µm-thick, frozen bovine placenta section acellularized with 0.5 % SDS and stained with hematoxylin-eosin. (The bar on the photograph corresponds to 200 µm.)
Fig. 8 is an optical microscopic photograph of a cell culture carrier of a 5 µm-thick, frozen bovine placenta section acellularized with 1.0 % SDS and stained with hematoxylin-eosin. (The bar on the photograph corresponds to 200 µm.)
Fig. 9 is a phase-contrast microscopic photograph of a one-day culture of BeWo cells on a slideglass with no tissue section stuck thereto. (The bar on the photograph corresponds to 200 µm.)
Fig. 10 is a fluorescent microscopic photograph of a one-day culture of BeWo cells on a slideglass with no tissue section stuck thereto, in which the living cells are identified by their calcein fluorescence. (The bar on the photograph corresponds to 200 µm.)
Fig. 11 is a phase-contrast microscopic photograph of a one-day culture of BeWo cells on a cell culture carrier of a 5 µm-thick, frozen bovine placenta section. (The bar on the photograph corresponds to 200 µm.)
Fig. 12 is a fluorescent microscopic photograph of a one-day culture of BeWo cells on a cell culture carrier of a 5 µm-thick, frozen bovine placenta section, in which the living cells are identified by their calcein fluorescence. (The bar on the photograph corresponds to 200 µm.)
Fig. 13 is a phase-contrast microscopic photograph of a one-day culture of BeWo cells on a cell culture carrier of a 5 µm-thick, frozen bovine placenta section acellularized with 0.1 % SDS. (The bar on the photograph corresponds to 200 µm.)
Fig. 14 is a fluorescent microscopic photograph of a one-day culture of BeWo cells on a cell culture carrier of a 5 µm-thick, frozen bovine placenta section acellularized with 0.1 % SDS, in which the living cells are identified by their calcein fluorescence. (The bar on the photograph corresponds to 200 µm.)
Fig. 15 is a phase-contrast microscopic photograph of a 4 -day culture of BeWo cells on a slideglass with no tissue section stuck thereto. (The bar on the photograph corresponds to 200 µm.)
Fig. 16 is a fluorescent microscopic photograph of a 4-day culture of BeWo cells on a slideglass with no tissue section stuck thereto, in which the living cells are identified by their calcein fluorescence. (The bar on the photograph corresponds to 200 µm.)
Fig. 17 is a phase-contrast microscopic photograph of a 4-day culture of BeWo cells on a cell culture carrier of a 5 µm-thick, frozen bovine placenta section. (The bar on the photograph corresponds to 200 µm.)
Fig. 18 is a fluorescent microscopic photograph of a 4-day culture of BeWo cells on a cell culture carrier of a 5 µm-thick, frozen bovine placenta section, in which the living cells are identified by their calcein fluorescence. (The bar on the photograph corresponds to 200 µm.)
Fig. 19 is a phase-contrast microscopic photograph of a 4-day culture of BeWo cells on a cell culture carrier of a 5 µm-thick, frozen bovine placenta section acellularizedwith 0.1% SDS. (The bar on the photograph corresponds to 200 µm.)
Fig. 20 is a fluorescent microscopic photograph of a 4 -day culture of BeWo cells on a cell culture carrier of a 5 µm- thick, frozen bovine placenta section acellularized with 0.1 % SDS, in which the living cells are identified by their calcein fluorescence. (The bar on the photograph corresponds to 200 µm.)
Fig. 21 is a phase-contrast microscopic photograph of a one-day culture of BeWo cells on a slideglass with no tissue section stuck thereto. (The bar on the photograph corresponds to 200 µm.)
Fig. 22 is a phase-contrast microscopic photograph of one-day culture of BeWo cells on a cell culture carrier of a 5 µm-thick, frozen bovine placenta section. (The bar on the photograph corresponds to 200 µm.)
Fig. 23 is a phase-contrast microscopic photograph of a one-day culture of BeWo cells on a cell culture carrier of a 10 µm-thick, frozen bovine placenta section. (The bar on the photograph corresponds to 200 µm.)
Fig. 24 is a phase-contrast microscopic photograph of a one-day culture of BeWo cells on a cell culture carrier of a 20 µm-thick, frozen bovine placenta section. (The bar on the photograph corresponds to 200 µm.)
Fig. 25 is a phase-contrast microscopic photograph of a one-day culture of BeWo cells on a cell culture carrier of a 5 µm-thick, frozen bovine placenta section acellularized with 0.1 % SDS. (The bar on the photograph corresponds to 200 µm.)
Fig. 26 is a phase-contrast microscopic photograph of a one-day culture of BeWo cells on a cell culture carrier of a 10 µm-thick, frozen bovine placenta section acellularized with 0.1 % SDS. (The bar on the photograph corresponds to 200 µm.)
Fig. 27 is a phase-contrast microscopic photograph of a one-day culture of BeWo cells on a cell culture carrier of a 20 µm-thick, frozen bovine placenta section acellularized with 0.1 % SDS. (The bar on the photograph corresponds to 200 µm.)
Fig. 28 is a phase-contrast microscopic photograph of NHDF cells cultured on a cell culture carrier of a 20 µm-thick, frozen bovine placenta section acellularized with 0.1 % SDS. As in this, the cells having proliferated in multilayers on the tissue section have begun to peel, like a sheet, from the slideglass on day 7 of the culture, while taking the section-derived tissue therein. (The bar on the photograph corresponds to 200 µm.)
Fig. 29 is a phase-contrast microscopic photograph of NHDF cells cultured on a cell culture carrier of a 20 µm-thick, frozen bovine placenta section acellularized with 0.1 % SDS, showing a part of the culture that differs from the part thereof shown by Fig. 28. As in this, the cells having proliferated in multilayers on the tissue section have begun to peel, like a sheet, from the slideglass on day 7 of the culture, while taking the section-derived tissue therein. (The bar on the photograph corresponds to 200 µm.)
Fig. 30 is a phase-contrast microscopic photograph of the NHDF cells having proliferated in multilayers on the 20 µm-thick section and completely peeled from the slideglass into the culture medium. (The bar on the photograph corresponds to 200 µm.)
Fig. 31 is a phase-contrast microscopic photograph of the cell sheet which has taken the section-derived tissue therein and which has been further cultured in a hydrophobic culture dish for 7 hours. (The bar on the photograph corresponds to 200 µm.)
Fig. 32 is a phase-contrast microscopic photograph of a three-dimensional multicellular aggregate with the section-derived tissue therein, which was formed by further culturing the cell sheet in the hydrophobic culture dish for 2 days. (The bar on the photograph corresponds to 200 µm.)
Fig. 33 is a phase-contrast microscopic photograph of a one-day culture of CPAE cells on a slideglass with no tissue section stuck thereto. (The bar on the photograph corresponds to 200 µm.)
Fig. 34 is a phase-contrast microscopic photograph of a one-day culture of CPAE cells on a cell culture carrier of a 5 µm-thick, frozen bovine placenta section. (The bar on the photograph corresponds to 200 µm.)
Fig. 35 is a phase-contrast microscopic photograph of a one-day culture of CPAE cells on a cell culture carrier of a 5 µm-thick, frozen bovine placenta section acellularized with 0.1 % SDS. (The bar on the photograph corresponds to 200 µm.)
Fig. 36 is a phase-contrast microscopic photograph of a 3 -day culture of CPAE cells on a slideglass with no tissue section stuck thereto. (The bar on the photograph corresponds to 200 µm.)
Fig. 37 is a phase-contrast microscopic photograph of a 3-day culture of CPAE cells on a cell culture carrier of a 5 µm-thick, frozen bovine placenta section. (The bar on the photograph corresponds to 200 µm.)
Fig. 38 is a phase-contrast microscopic photograph of a 3-day culture of CPAE cells on a cell culture carrier of a 5 µm-thick, frozenbovine placenta section a cellularized with 0.1% SDS. (The bar on the photograph corresponds to 200 µm.)
Fig. 39 is an optical microscopic photograph of a 3-day culture of CPAE cells on a cell culture carrier of a 5 µm-thick, frozen bovine placenta section, which was stained with hematoxylin-eosin. (The bar on the photograph corresponds to 200 µm.)
Fig. 40 is an optical microscopic photograph of a 3-day culture of CPAE cells on a cell culture carrier of a 5 µm-thick, frozen bovine placenta section acellularized with 0.1 % SDS, which was stained with hematoxylin-eosin. (The bar on the photograph corresponds to 200 µm.)
Fig. 41 is an optical microscopic photograph of a 2-day culture of PC-12 cells on a cell culture carrier of a 5 µm-thick, frozen bovine placenta section, which was stained with hematoxylin-eosin. This especially shows the region corresponding to the cotyledon around the placental septa of the bovine placenta section. (The bar on the photograph corresponds to 200 µm.)
Fig. 42 is an optical microscopic photograph of a 2-day culture of PC-12 cells on a cell culture carrier of a 10 µm-thick, frozen bovine placenta section, which was stained with hematoxylin-eosin. This especially shows the region corresponding to the cotyledon around the placental septa of the bovine placenta section. (The bar on the photograph corresponds to 200 µm.)
Fig. 43 is an optical microscopic photograph of a 2-day culture of PC-12 cells on a cell culture carrier of a 20 µm-thick, frozen bovine placenta section, which was stained with hematoxylin-eosin. This especially shows the region corresponding to the cotyledon around the placental septa of the bovine placenta section. (The bar on the photograph corresponds to 200 µm.)
Fig. 44 is an optical microscopic photograph of a 2-day culture of PC-12 cells on a slideglass with no tissue section stuck thereto, which was stained with hematoxylin-eosin. (The bar on the photograph corresponds to 200 µm.)
Fig. 45 is an optical microscopic photograph of a 2-day culture of PC-12 cells on a cell culture carrier of a 10 µm-thick, frozen bovine placenta section, which was stained with hematoxylin-eosin. This especially shows the region which is rich in the connective tissue of the caruncle adjacent to the cotyledon in the bovine placenta section. (The bar on the photograph corresponds to 200 µm.)
Fig. 46 is an optical microscopic photograph of a 2-day culture of PC-12 cells on a cell culture carrier of a 10 µm-thick, frozen bovine placenta section, which was stained with hematoxylin-eosin. This especially shows the region on the side of the lamina propria mucosae of the caruncle in the bovine placenta section. (The bar on the photograph corresponds to 200 µm.)
Fig. 47 is a phase-contrast microscopic photograph of a 5-day serum-free culture of PC-12 cells on a slideglass with no tissue section stuck thereto. (The bar on the photograph corresponds to 200 µm.)
Fig. 48 is a phase-contrast microscopic photograph of a 5-day serum-free culture of PC-12 cells on a cell culture carrier of a 5 µm-thick, frozen bovine placenta section acellularized with 0.1 % SDS. (The bar on the photograph corresponds to 200 µm.)
Fig. 49 is a phase-contrast microscopic photograph of a 5-day serum-free culture of PC-12 cells on a cell culture carrier of a 5 µm-thick, frozen bovine placenta section. (The bar on the photograph corresponds to 200, µm.)
Fig. 50 is an optical microscopic photograph of a 5-day serum-free culture of PC-12 cells on a cell culture carrier of a 5 µm- thick, frozen bovine placenta section acellularized with 0.1 % SDS, which was stained with hematoxylin-eosin. (The bar on the photograph corresponds to 200 µm.)
Fig. 51 is an optical microscopic photograph of a 5-day serum-free culture of PC-12 cells on a cell culture carrier of a 5 µm-thick, frozen bovine placenta section, which was stained with hematoxylin-eosin. (The bar on the photograph corresponds to 200 µm.)

### BEST MODE FOR CARRYING OUT THE INVENTION

The invention of this application is characterized by the above-mentioned features, and its embodiments are described below.

The living tissue to be used in the invention of this application may be a part or all of an unborn non-human or born animal, or a part or all of a plant. In addition, it may also be a tissue previously fixed with a fixing reagent, or a fresh tissue. The fixing reagent includes, for example, methanol, ethanol, acetone, formalin, glutaraldehyde. Mammals for the living tissue include, for example, human, monkey, bovine, ovine, goat, baboon, porcine, dog, rabbit, guinea pig, hamster, rat, and mouse. The animal tissue for use herein is not specifically defined, and may be a part or all of an animal. For example, it includes brain, spinal cord, muscle, skin, blood vessel, gullet, stomach, small intestine, large intestine, heart, lung, liver, kidney, pancreas, spleen, thymus, bone, cartilage, bone marrow, uterus, ovary, oviduct, testis, placenta, and umbilical cord. Their normal tissues as well as focal tissues such as tumors are usable herein.

The tissue sections for use in the invention may be frozen sections, or may also be paraffin-embedded sections or resin-embedded sections. In the field of histology and pathology, well employed are experimental methods of preparing sections of a part or all of an animal or plant tissue, spreading the section on a slideglass, staining it thereon, and observing it with a microscope. Needless-to-say, such tissue sections include all the site and time-dependent information on the living tissue, and the information can be analyzed not only through general staining but also through immunohistochemical technology or in-situ hybridization.

According to the technology, the tissue sections may be treated with an antibody or a nucleic acid probe capable of binding a physiologically-active agent to thereby introduce an exogenous physiologically-active agent into a specific site of the sections. The physiologically-active agent includes, for example, proteins, glycoproteins, lipoproteins, peptides, saccharides, lipids, liposaccharides, nucleic acids, and low-molecular compounds. The activities of such physiologically-active agents are, for example, antibody activities, enzymatic activities, hormonal activities, cytokine activities, cell adhesion factors, growth factors, differentiation factors, regeneration factors, and morphogenesis factors.

In addition, the tissue sections may be biologically treated with, for example, enzyme, or chemically treated with, for example, acid, alkali or surfactant to thereby change or modify the constituent components and/or the microstructure of the sections. Also in the invention of this application, the frozen tissue sections to be used may be prepared, for example, by rapidly freezing an unfixed or formalin-fixed tissue with liquid nitrogen or the like, either directly as it is or after having been embedded in an OCT compound, and then cutting it with a cryomicrotome into sections having a thickness of from 1 to 50 µm, preferably from 4 to 20 µm. The cryosections of an unfixed tissue may be fixed with formalin or the like. Paraffin-embedded tissue sections are prepared generally by fixing a tissue with formalin or the like, dehydrating it with ethanol, substituting it with xylene or paraffin, embedding it in paraffin, and then cutting it with a microtome into sections having a thickness of from 0.5 to 50 µm, preferably from 2 to 20 µm. Resin-embedded tissue sections are prepared generally by fixing a tissue with formalin or the like, dehydrating it with ethanol, embedding it in resin such as historesin, and then cutting it with a microtome into sections having a thickness of from 0.1 to 50 µm, preferably from 0.5 to 10 µm.

Before or after sectioned, the tissue for use in the invention may be acellularized. For acellularizing it, for example, used is a solution of any one of salts, alkalis, surfactants, chelating agents and enzymes, or their combinations. The salts include, for example, sodium chloride, magnesium chloride, and potassium phosphate; and the alkalis include, for example, sodium hydroxide, calcium hydroxide, and ammonia. The surfactants includes, for example, sodium dodecyl sulfate, Triton X-100, Tween 20 and 80. The chelating agents include, for example, EDTA, and BGTA. The enzymes include, for example, proteases such as trypsin, collagenase, disperse, elastase, papain, matrix metalloprotease; saccharide-degradable enzymes such as hyaluronidase; and nucleases such as deoxyribonuclease.

The culture carrier of a tissue section to be used in this invention may be a tissue section alone or may be a composite structure of a tissue section stuck to a support or stuck thereto while stretched. The support includes, for example, glass, plastics, rubber, metal, natural or synthetic thread and/or its woven matters, and biodegradable materials. If desired, the support may be previously processed for promoting the section adhesion thereto or for promoting the section adhesion thereto under stretch. For the treatment to promote the section adhesion thereto or to promote the section adhesion thereto under stretch, the support may be coated with, for example, polylysine or APS. In case where the tissue section to be the culture carrier contains OCT compound, paraffin or resin, it may be washed with water, or may be processed for paraffin removal or resin removal from it. Also if desired, the tissue section to be the culture carrier may be sterilized by processing it with ethanol, ethylene oxide gas, g-rays or ultraviolet rays.

The animal cells to be cultured in this invention may be any of primary culture cells, cells of established cell lines, non-human blastocysts, or cells with an exogenous gene introduced thereinto. One or more different types of cells may be cultured on the culture carrier of this invention.

The animal cells may be derived from any of undifferentiated stem cells, cells under differentiation, terminally differentiated cells and/or dedifferentiated cells, wherein the cells are not human embyryonic cells. The undifferentiated stem cells may be especially non-human embryonic stem cells. To start the culture of such animal cells, cell suspensions, tissue explants, non-human blastocysts, or three-dimensionally reconstructed multicellular aggregates are seeded on the carrier. The liquid medium for the culture of the animal cells may be any and every one generally used for ordinary animal cell culture, and its basic composition may be any and every ordinary one. It may or may not contain any additive of serum, antibiotics, vitamins, etc. A suitable support for the culture carrier is selected so that the affinity between the cultured animal cells and the tissue section on the support is larger than that between the tissue section and the support. In that condition, the section or the section-derived tissue on which the animal cells have adhered and proliferated is easy to peel from the support. In addition, if the section or the section-derived tissue with the proliferated cells is further cultured after peeled from the support, three-dimensional multicellular aggregates that have involved the section or the section-derived tissue therein can be formed.

Animal cells can be cultured with ease on the culture carrier of this invention, for example, as follows: One or more 6 µm-thick, frozen animal tissue sections are stuck to and stretched on a slideglass, and then suitably processed thereon for fixation, acellularization, sterilization and/or equilibration with a culture medium; then the slideglass is set in a culture dish; and animal cells in suspension are seeded on the culture carrier in the dish. In that condition, the animals cells readily grow on the carrier. The animal cells having been thus cultured on the culture carrier of the invention can be transplanted with ease, for example, as follows: A 6 µm-thick, frozen animal tissue section is stuck to a biodegradable mesh sheet; then this is suitably processed for fixation, acellularization, sterilization and/or equilibration with a culture medium, and set in a culture dish; then animal cells in suspension are seeded to grow on the culture carrier in the dish; and the culture carrier with the animal cells having grown thereon is transplanted onto a desired organ.

### EXAMPLES

### (Example 1: Preparation of animal tissue section-containing cell culture carrier)

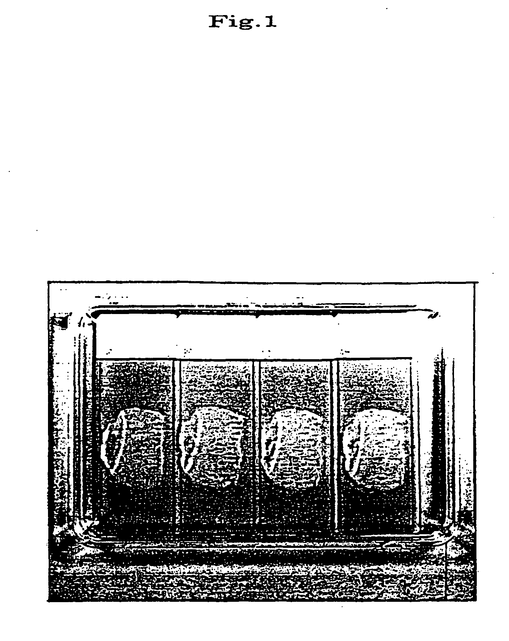

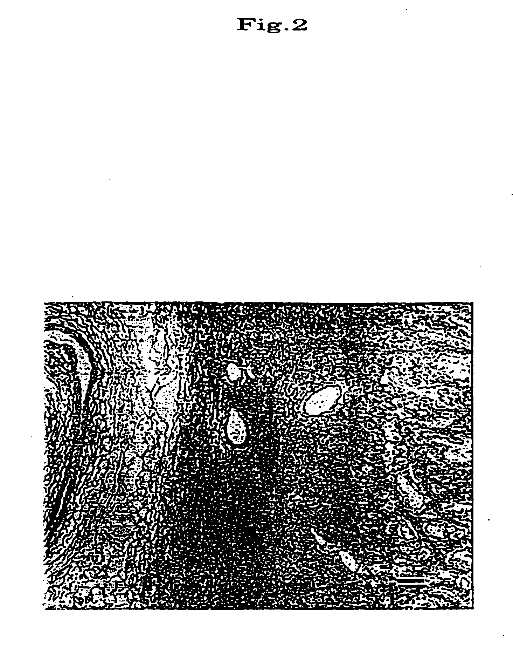

The bovine placenta taken out of a slaughtered 241-day pregnant cow was embedded in an OCT compound, and then rapidly frozen with liquid nitrogen. Using a cryomicrotome, this was sectioned into sections with a thickness of 5, 10 and 20-µm. Each section was put on a slideglass (Matsunami Glass Industry's S-0317). The operation after this was effected in a clean bench. Four slideglasss with the frozen section stuck to each under stretch were set in a culture tray (Sumitomo Bakelite's MS-3300N) (see Fig. 1). 20 ml of HBSS (Hanks' balanced salt solution; GIBCO BRL #14025-092) was poured into the tray, kept static at room temperature for 5 minutes, and then removed. Through this operation, the OCT compound was removed from the tissue section. Next, 20 ml of 70 % ethanol was poured into the tray, kept static at room temperature for 10 minutes, and then removed. Through this operation, the tissue section was fixed and sterilized. Next, the slideglasss in the tray were rinsed twice with 20 ml of HBSS and once with 20 ml of the culture medium to be used in this experiment. In that manner, a cell culture carrier of the frozen bovine placenta section, stuck to the slideglass serving as a support, was prepared in the culture tray. The tray serves as a culture vessel in the following process. The cell culture carrier was stained with hematoxylin-eosin in a standard procedure, and observed. As a result, the cell culture carrier was identified as the bovine placenta tissue (see Fig. 2).

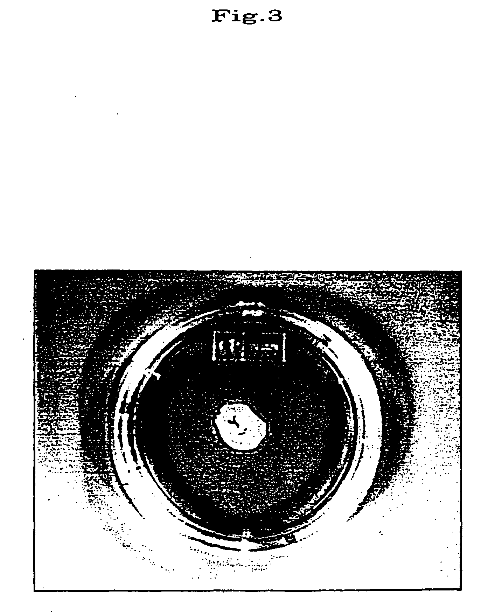

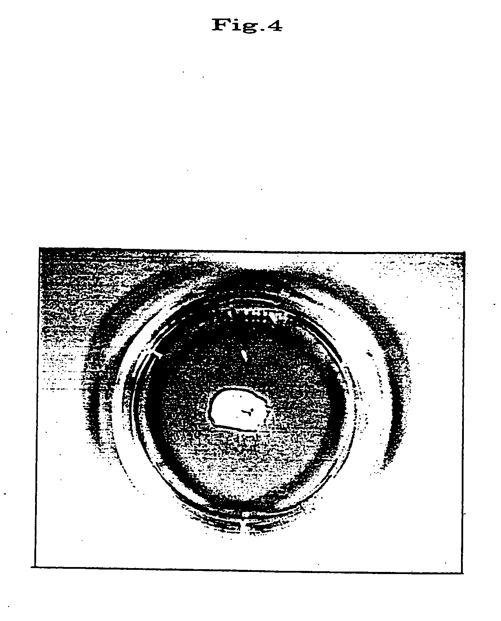

On the other hand, the bovine uterus that had been taken out of a slaughtered 13-day estrous nulliparous cow was fixed with a 10 % formalin neutral buffer, and then embedded in paraffin in a standard procedure known in the art of pathohistology. This was sectioned with a microtome into 5 µm-thick sections. While stretched, the section was stuck to a slideglass (Matsunami Glass Industry's S-0317). This was processed with xylene for paraffin removal from it, and then processed with ethanol of which the concentration decreased from 100 % to 50 % in order. The operation after this was effected in a clean bench. Every slideglass with the tissue section stuck thereto was inserted into an individual 100-mmϕ culture dish (Falcon #351001). 10 ml of HBSS was poured into the dish, and gently stirred at room temperature for 5 minutes to thereby peel the tissue section from the slideglass. The thus-peeled tissue section was transferred into a 35-mmφ hydrophilic culture dish (Falcon #353001) or hydrophobic culture dish (Falcon #351008) of polystyrene filled with 2 ml of sterilized water, along with a small amount of HBSS put thereinto with a pipette. One tissue section was in one dish. In every dish, the tissue section was rinsed twice with.2 ml of sterilized water. In the hydrophilic culture dish, the tissue section was, while stretched thereon, dewatered as much as possible and then dried in air. In that manner, a cell culture carrier derived from the paraffin-embedded, bovine uterus section was prepared, stuck to the 35-mmφ hydrophilic culture dish of polystyrene serving as a support (see Fig. 3). On the other hand, the hydrophobic culture dish was, after rinsed twice with sterilized water, further rinsed once with 2 ml of 70 % ethanol. Then, the tissue section in this was, while stretched thereon, processed to remove the 70 % ethanol as much as possible, and then dried in air. In that manner, a cell culture carrier derived from the paraffin-embedded, bovine uterus section was prepared, stuck to the 35-mmφ hydrophobic culture dish of polystyrene serving as a support (see Fig. 4).

### (Example 2: Preparation of acellularized tissue section-containing cell culture carrier)

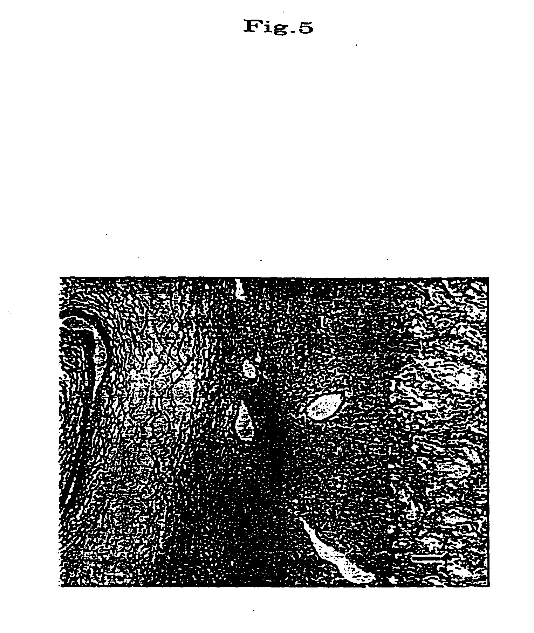

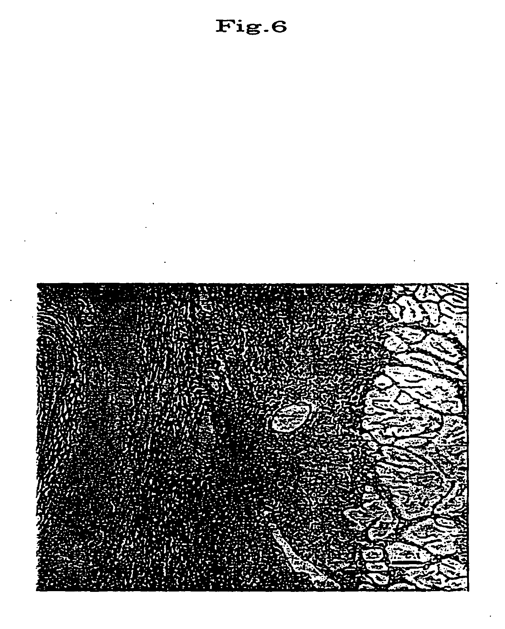

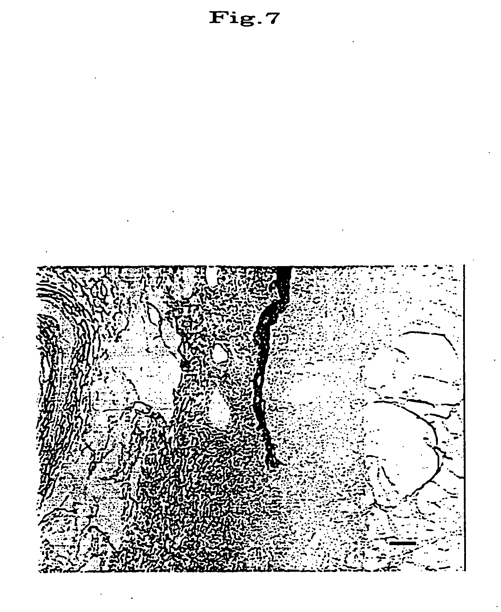

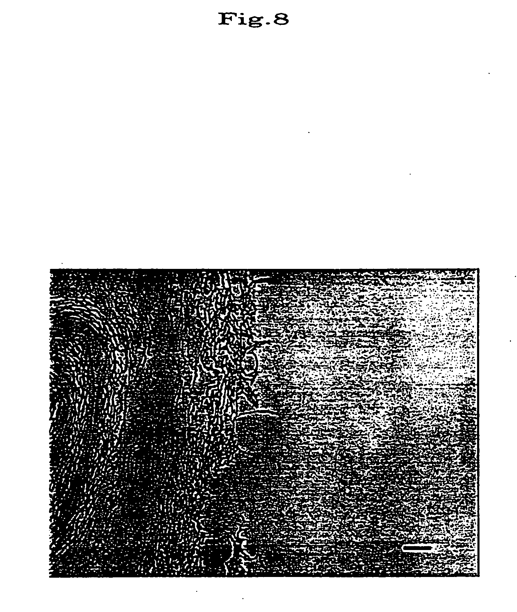

In the same manner as in Example 1, slideglasss with a frozen bovine placenta section stuck to each under stretch were set in a culture tray in a clean bench, and 20 ml of HBSS was poured into the tray and kept static at room temperature for 5 minutes to remove the OCT compound from each tissue section. Next, every slideglass with the tissue section stuck thereto was transferred from the tray into a individual 100-mmϕ culture dish (Falcon #351001). 10 ml of 0.01, 0.1, 0.5 or 1.0 % SDS (sodium dodecyl sulfate) was poured into each dish, kept static at room temperature for 10 minutes, and then removed. Through this operation, acellularizing the tissue sections was investigated. Next, the tissue section-stuck slideglass in each dish was rinsed twice with 20 ml of HBSS and then once with 10 ml of the culture medium to be used in this experiment. Then, each tissue section was stained with hematoxylin-eosin in a standard procedure, and observed. This confirmed that the frozen bovine placenta section processed with 0.1 % SDS was acellularized (see Figs. 5 to 8). In that manner, a cell culture carrier of the acellularized, frozen bovine placenta section was prepared, stuck to the slide serving as a support.

### (Example 3: Culture of BeWo cells on cell culture carrier of frozen bovine placenta section)

BeWo cells of an established, human choriocarcinoma cell line were tried on the cell culture carrier of the bovine placenta section, in point of the toxicity of the carrier, the influence of the carrier on the cell adhesion, spread and proliferation, and the influence thereof on the cell morphology depending on the thickness of the tissue section. The BeWo cells (JCRB9111, obtained from the Japan Health Sciences Foundation) were subcultured in a culture medium, Ham's F12 (GIBCO BRL #11765-054) containing 15 % heat-inactivated fetal bovine serum (SIGMA #F-2442), 100 units/ml penicillin and 100 µg/ml streptomycin (GIBCO BRL #15140-148).

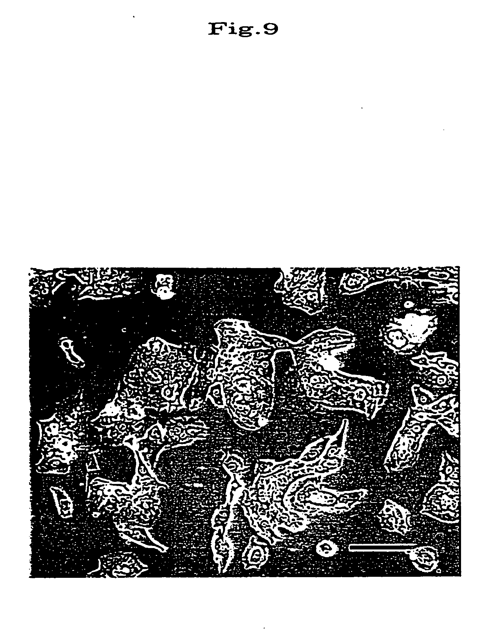

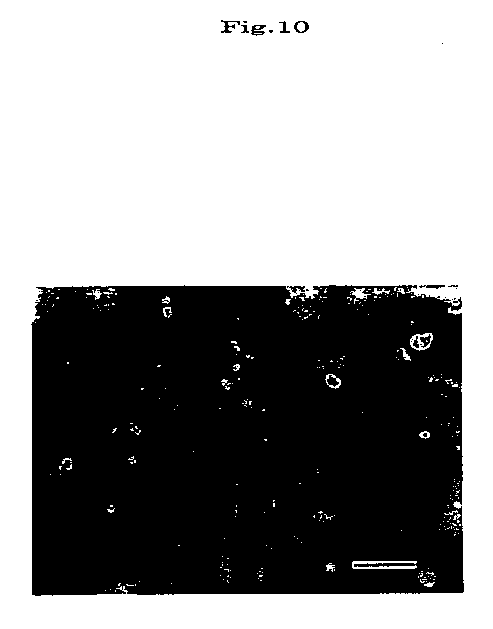

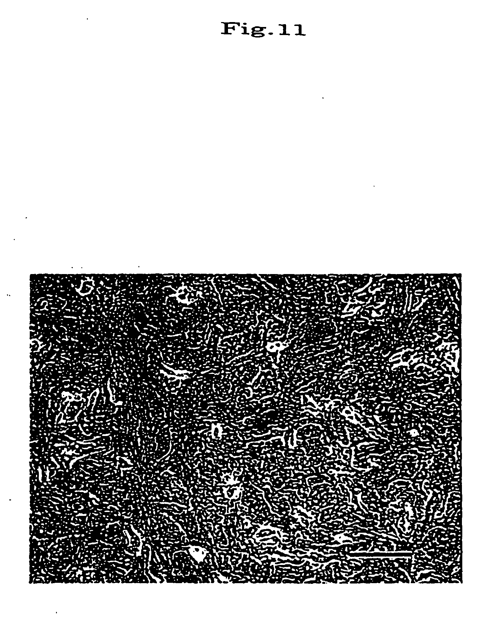

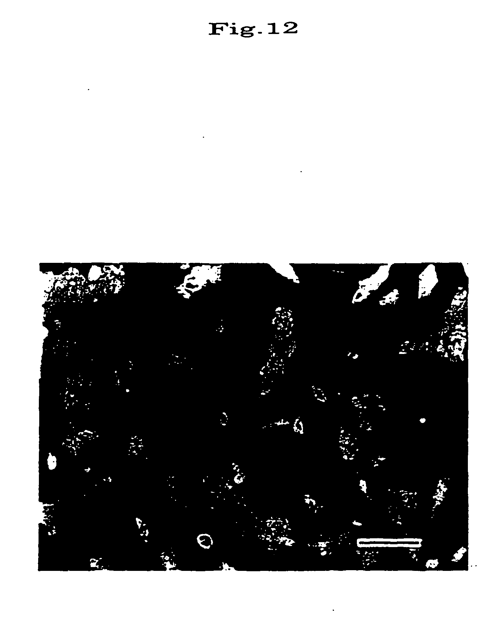

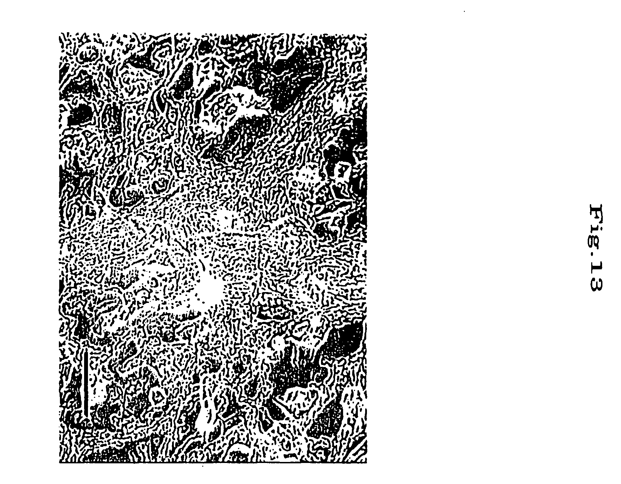

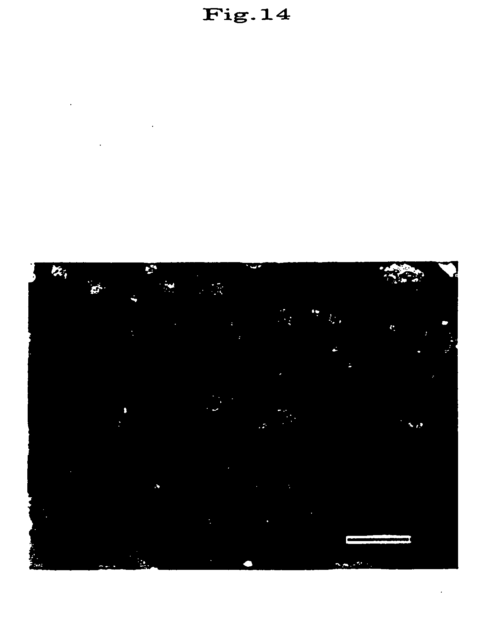

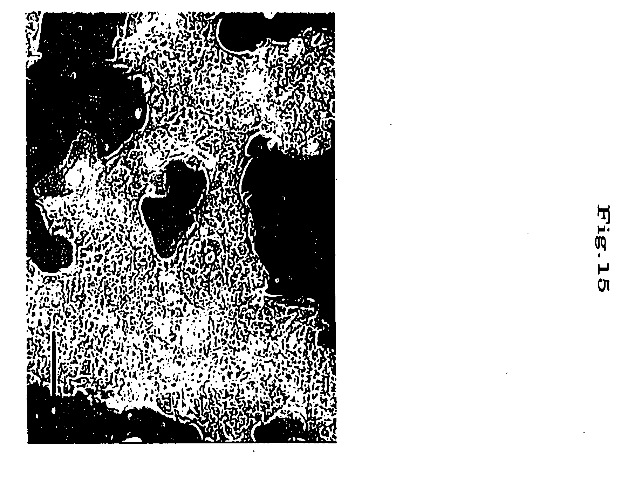

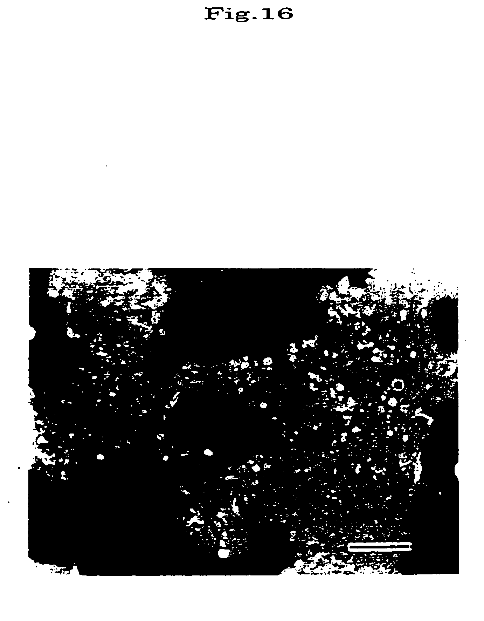

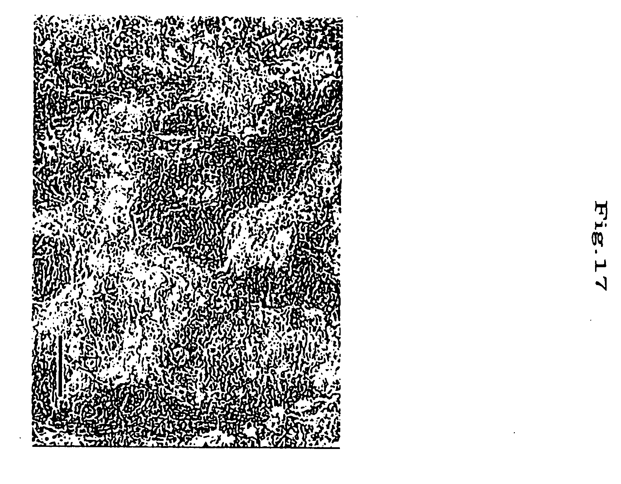

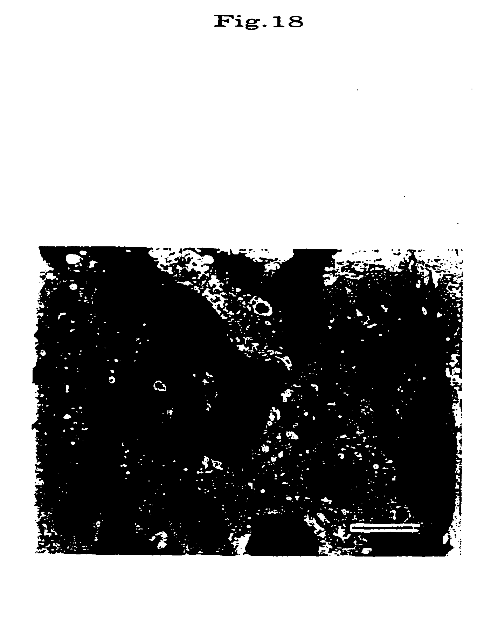

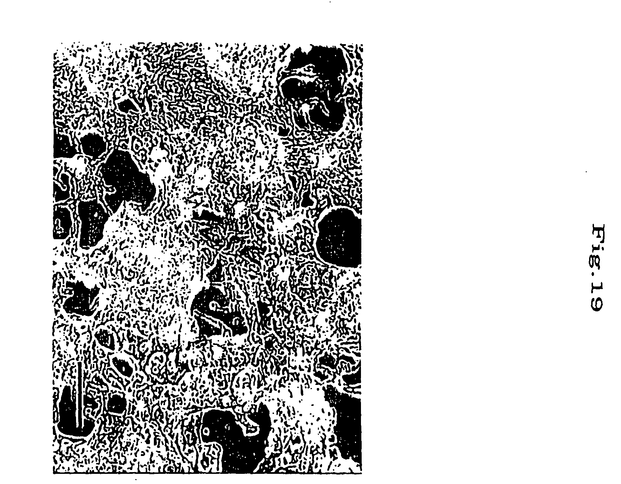

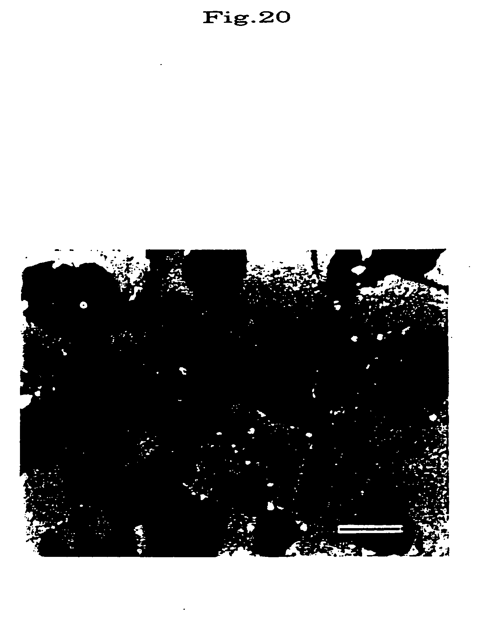

Two slideglasss each having thereon the cell culture carrier of 5 µm-thick frozen bovine placenta section that had been prepared in Example 1, and two slideglases each having thereon the cell culture carrier of 5 µm- thick frozen bovine placenta section acellularized with 0.1 % SDS that had been prepared in Example 2, totaling four, were inserted into culture tray. The BeWo cells suspended in 20 ml of the culture medium were seeded in the tray to have a final cell density of 2.1 × 10⁴ cells/cm², and cultured in a humidified incubator containing 5 % CO₂ and 95 % air at 37°C. On day 1 of the culture, every slideglass was transferred from the tray into an individual 100-mmϕ culture dish filled with 10 ml of the culture medium. After this, the cells were cultured for 4 days with changing the culture medium everyday. On day 1 and day 4 of the culture, the cells were observed with a phase-contrast microscope for their morphology; and based on the calcein AM (Molecular Probes #L-3224) metabolism of the living cells, they were observed with a fluorescent microscope for their calcein fluorescence. Concretely, the cultured cells were observed for their calcein AM metabolism as follows: The culture medium was removed from the dish, and the cells were rinsed twice with 10 ml of PBS, and 10 ml of HBSS containing 2 µM calcein AM was added thereto. In that condition, the cells were cultured in a humidified incubator containing 5 % CO₂ and 95 % air at 37°C for 15 minutes. Then, the thus-cultured cells were observed with a fluorescent microscope. As a result, it was found that the cells adhered and spread on day 1 of the culture and proliferated on day 4 of the culture on both the tissue section carrier and the acellularized tissue section carrier, like on the slideglass with no section thereon, and that the thus-adhered, spread and proliferated cells were all alive on every carrier (see Figs. 9 to 20). The result suggests that both the cell culture carrier of the tissue section and that of the acellularized tissue section have no cytotoxicity.

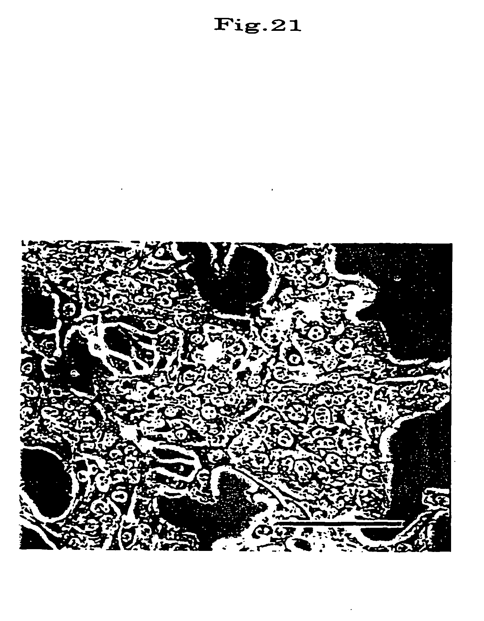

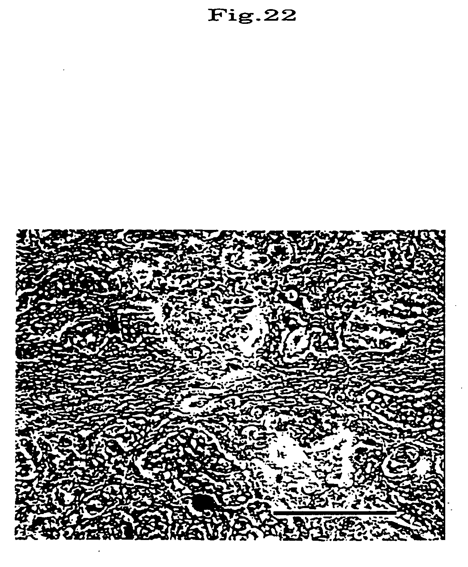

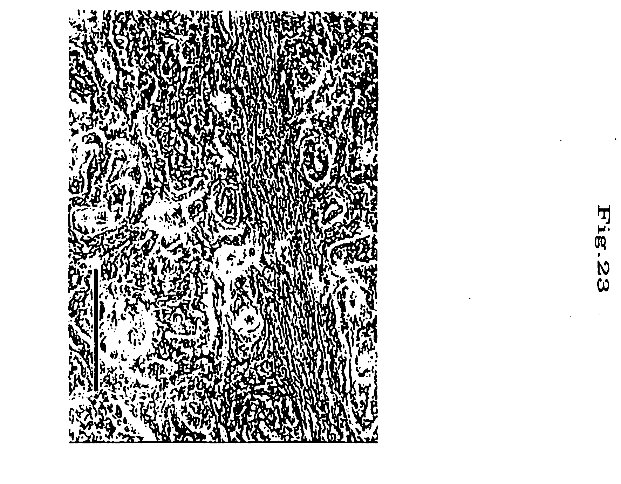

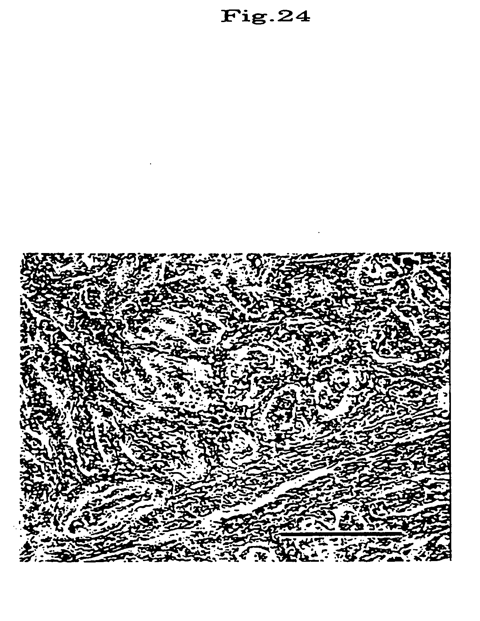

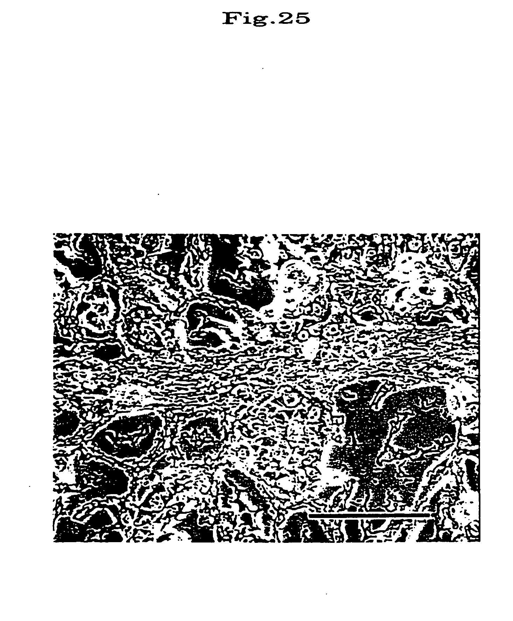

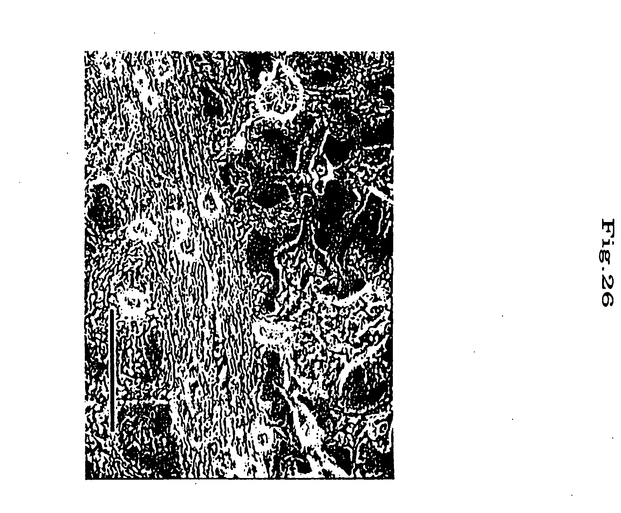

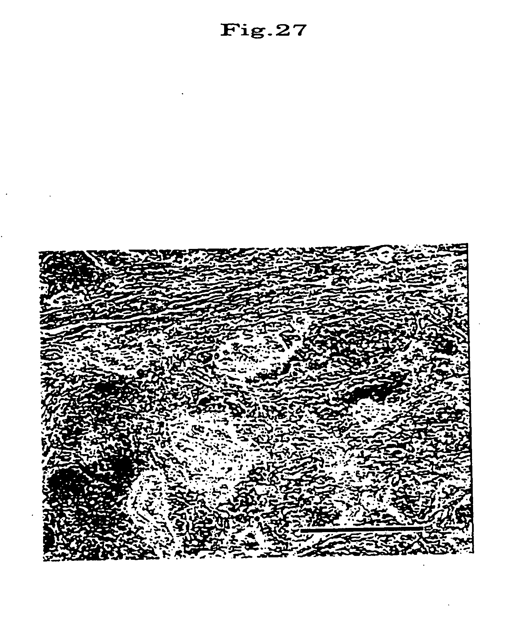

Next, the cell culture carriers of 5, 10 or 20 µm-thick frozen bovine placenta tissue section prepared on slideglasss in Example 1, and the cell culture carriers of 5, 10 or 20 µm- thick frozen bovine placenta tissue section acellularized with 0.1 % SDS and prepared on slideglases in Example 2 were inserted into a culture tray. The BeWo cells suspended in 20 ml of the culture medium were seeded in the tray to have a final cell density of 4.5 × 10⁴ cells/cm², and cultured in a humidified incubator containing 5 % CO₂ and 95 % air at 37°C for 1 day. The cultured cells were observed with a phase-contrast microscope for their morphology. It was found that, on the slideglass with no tissue section thereon, the cells in the adhered colonies well spread like cobblestones, but on the tissue section and on the acellularized tissue section, the cells in the adhered colonies tended to aggregate in rounds with the increase in the thickness of the sections (see Figs. 21 to 27).

### (Example 4: Culture of NHDF cells on cell culture carrier of frozen bovine placenta section)

Normal neonatal human dermalfibroblasts, NHDF cells were investigated for their ability to form three-dimensional multicellular aggregates taking therein the cell culture carrier of tissue section used for culturing the cells. The NHDF cells (Kurabo Industry's KF-4109) were subcultured in a culture medium, DMEM (Dulbecco's modified Eagle medium; GIBCO BRL #11885-084) containing 10 % heat-inactivated fetal bovine serum, 20 mM RBPES (GIBCO BRL #15630-080), 100 units/ml penicillin and 100 µg/ml streptomycin.

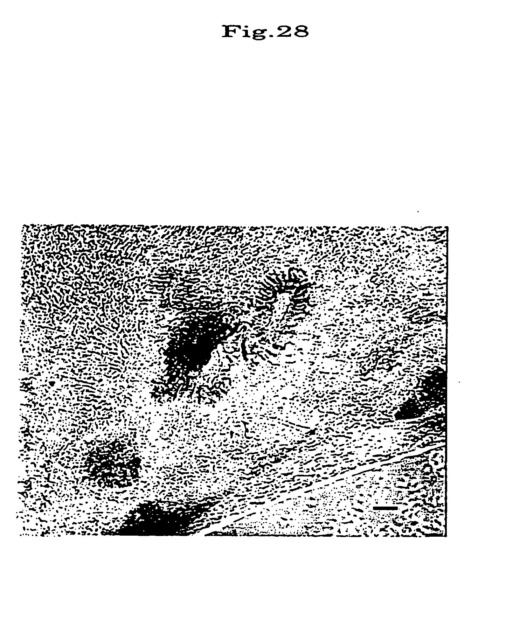

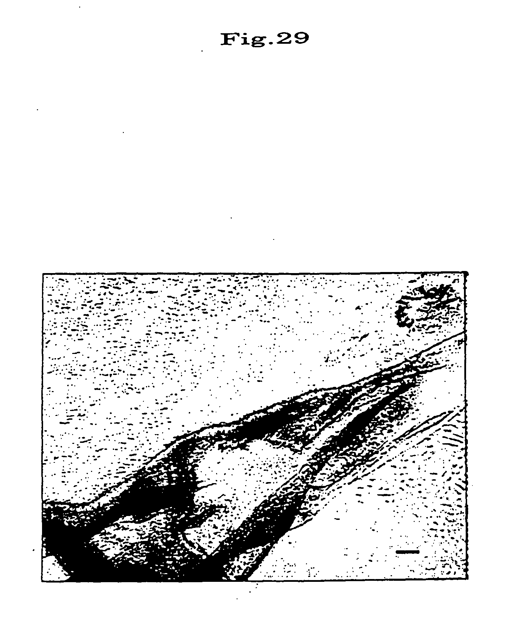

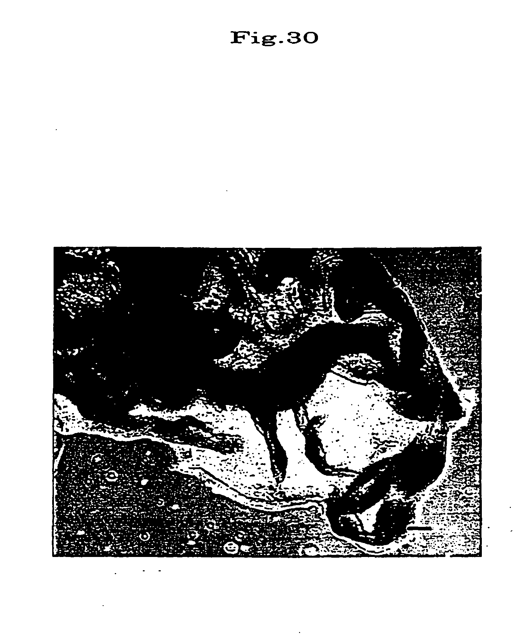

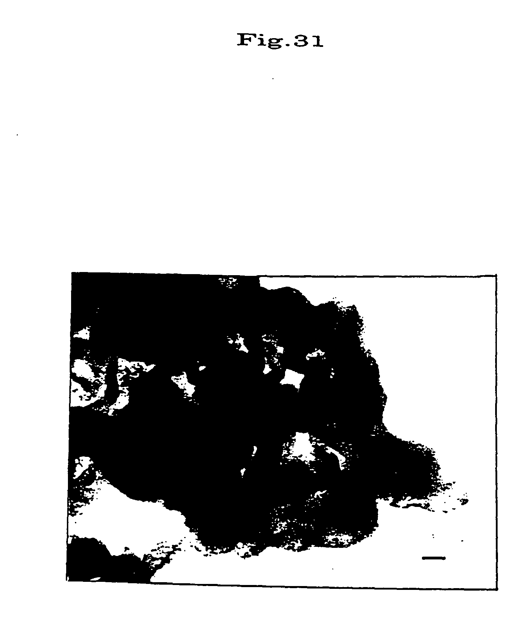

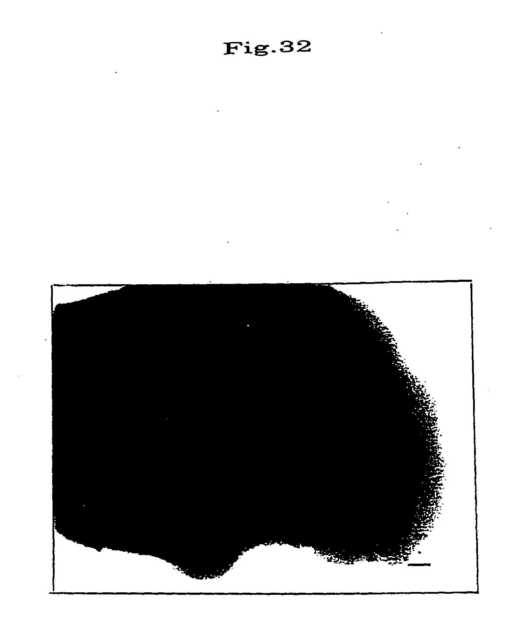

The slideglases each having thereon the cell culture carrier of 5, 10 or 20 µm-thick frozen bovine placenta section that had been prepared in Example 1, and the slideglases each having thereon the cell culture carrier of 5, 10 or 20 µm-thick frozen bovine placenta section acellularized with 0.1 % SDS that had been prepared in Example 2 were inserted into a culture tray. The NHDF cells suspended in 20 ml of the culture medium were seeded in the tray to have a final cell density of 3.3 × 10⁴ cells/cm², and cultured in a humidified incubator containing 5 % CO₂ and 95 % air at 37°C. Four hours after the start of the culture, every slideglass was transferred from the tray into an individual 100-mmφ culture dish filled with 10 ml of the culture medium. After this, the cells were cultured for 9 days with changing the culture medium every other day. The cells were observed with a phase-contrast microscope for their morphology. On day 7 of the culture, the cells having grown in multilayers on the 20 µm-thick acellularized section began to peel from the slideglass, forming a sheet with the section-derived tissue taking therein (see Figs. 28, 29). The cell sheet was completely peeled from the slideglass by gently pipetting it, and floated in the culture medium (see Fig. 30). The peeled cell sheet was transferred into a 35-mmφ hydrophobic culture dish of polystyrene, on which it is said that the cells hardly adhere, along with 2 ml of the culture medium thereinto, and further cultured in the dish. Seven hours after the start of the culture, the cell sheet having taken therein the section-derived tissue almost aggregated in the hydrophobic culture dish (see Fig. 31); and on day 2 of the culture(that is, on day 9 of the culture), it formed three-dimensional multicellular aggregate having a smooth surface (see Fig. 32). On day 8 of the culture, the cells having proliferated in multilayers on the 10 µm-thick acellularized section began to peel from the slideglass; and on day 9 of the culture, the other cells having proliferated in multilayers on the 5, 10 and 20 µm-thick sections and on the 5 µm-thick acellularized section began to peel from the slideglases.

### (Example 5: Culture of CPAE cells on cell culture carrier of frozen bovine placenta section)

CPAE cells of an established, bovine pulmonary artery endothelial cell line were tried on the cell culture carrier of tissue section to check their morphogenesis thereon. The CPAE cells (JCRB9022, obtained from the Japan Health Sciences Foundation) were subcultured in a culture medium, DMEM containing 10 % heat-inactivated fetal bovine serum, 20 mM HEPES, 100 units/ml penicillin and 100 µg/al streptomycin.

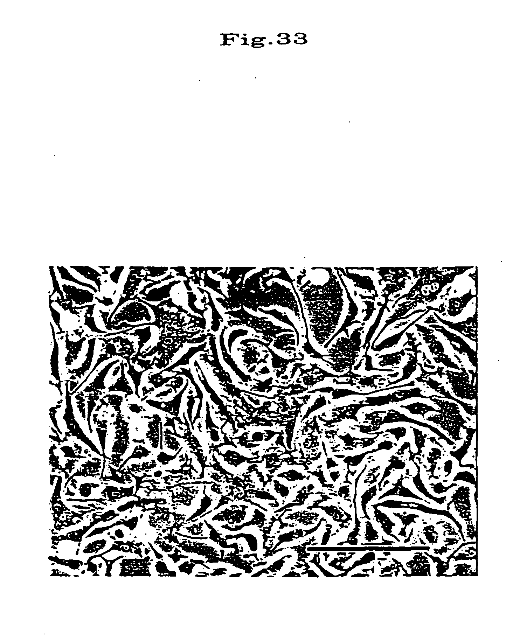

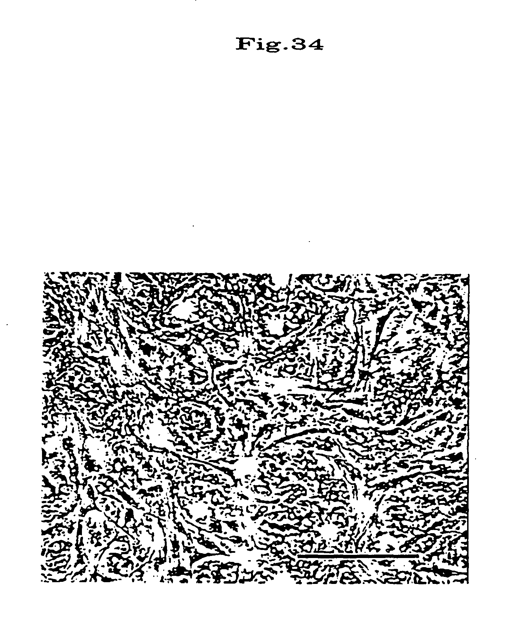

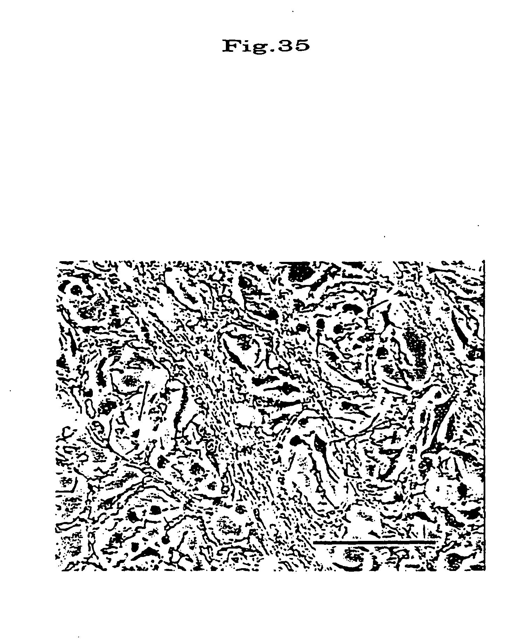

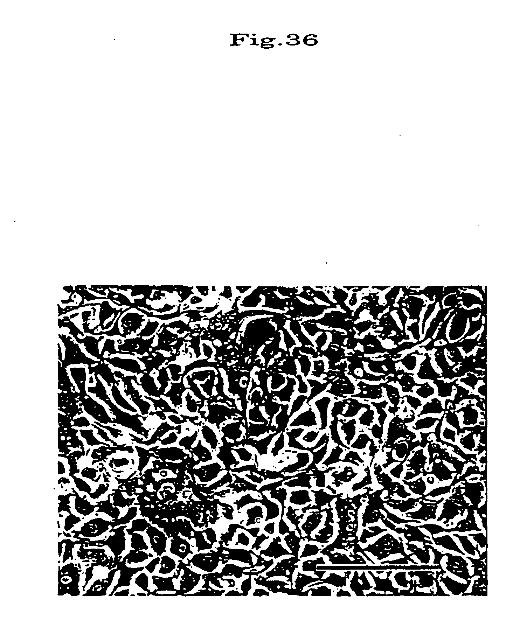

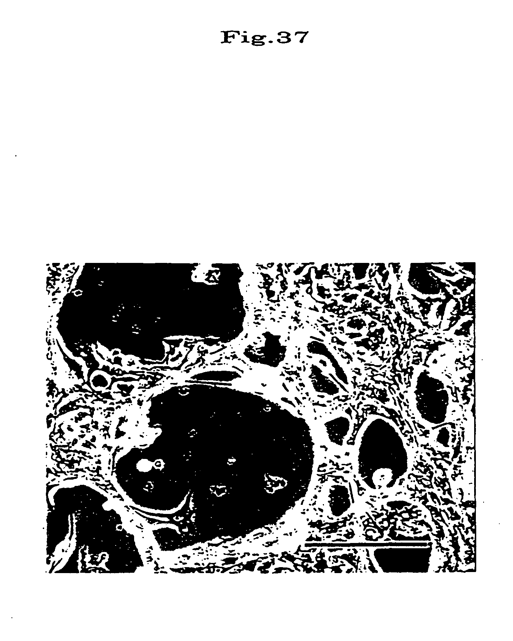

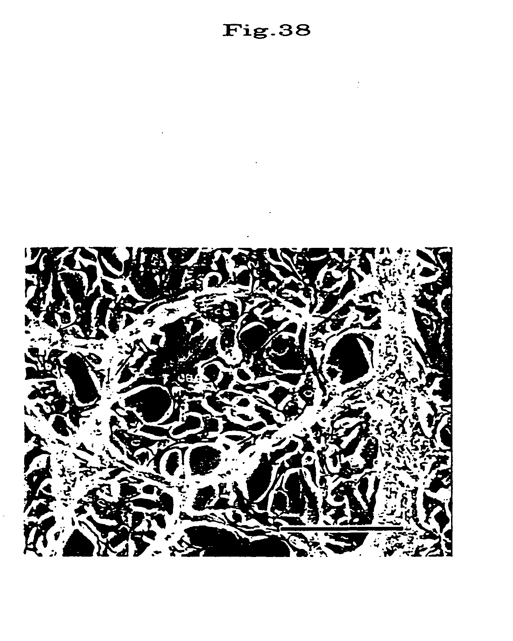

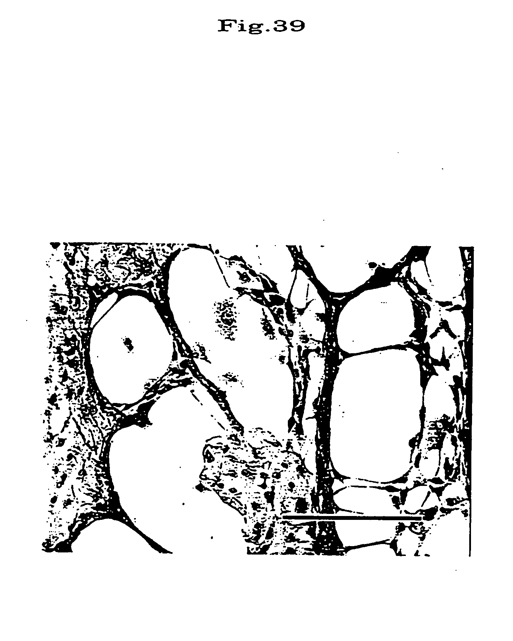

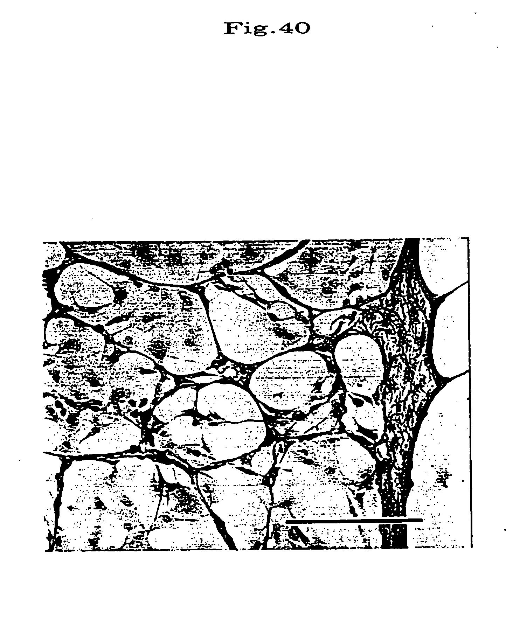

The slideglases each having thereon the cell culture carrier of 5 µm-thick frozen bovine placenta section that had been prepared in Example 1, and the slideglases each having thereon the cell culture carrier of 5 µm-thick frozen bovine placenta section acellularized with 0.1 % SDS that had been prepared in Example 2 were inserted into a culture tray. The CPAE cells suspended in 20 ml of the culture medium were seeded in the tray to have a final cell density of 3.4 × 10⁴ cells/cm², and cultured in a humidified incubator containing 5 % CO₂ and 95 % air at 37°C. One day after the start of the culture, every slideglass was transferred from the tray into an individual 100-mmϕ culture dish filled with 10 ml of the culture medium. After this, the cells were cultured for 3 days with changing the culture medium every other day. On day 1 of the culture, the cells were observed with a phase-contrast microscope for their morphology. It was found that, on the slideglass with no tissue section thereon, many cells adhered like cobblestones, but on the tissue section and on the acellularized tissue section, many cells elongated relatively thin (see Figs. 33 to 35). On day 3 of the culture, the cells were again observed with a phase-contract microscope for their morphology, and, stained with hematoxylin-eosin, they were observed with an optical microscope. It was found that, on the slideglass with no tissue section thereon, most cells adhered like cobblestones, but on the tissue section and on the acellularized tissue section, most cells formed a capillary network-like structure, having taken the section-derived tissue therein (see Figs. 36 to 40). (Example 6: Culture of PC-12 cells on cell culture carrier of frozen bovine placenta section)

PC-12 cells of rat pheochromocytoma were tried on the cell culture carrier of tissue section to check their morphogenesis thereon, on which the cells were cultured in both an ordinary subculture medium and a serum-free culture medium. The PC-12 cells (RCB0009, obtained from the Cell Bank of the Riken Gene Bank the Institute of Physical and Chemical Research) were subcultured in a culture medium, DMEM containing 10 % heat-inactivated fetal bovine serum, 20 mM HEPES, 100 units/ml penicillin and ,100 µg/ml streptomycin.

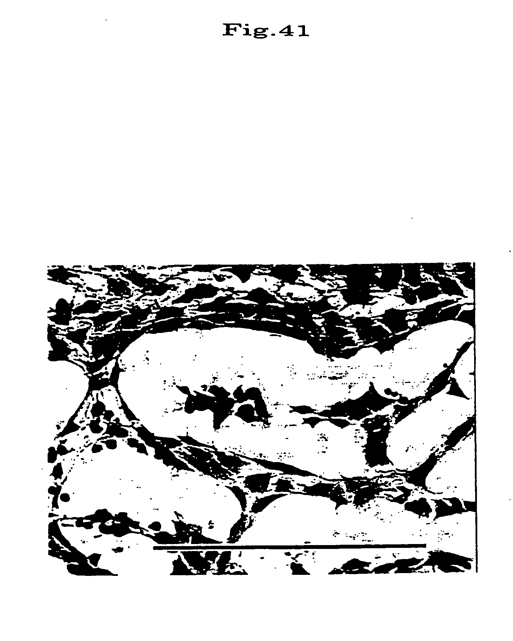

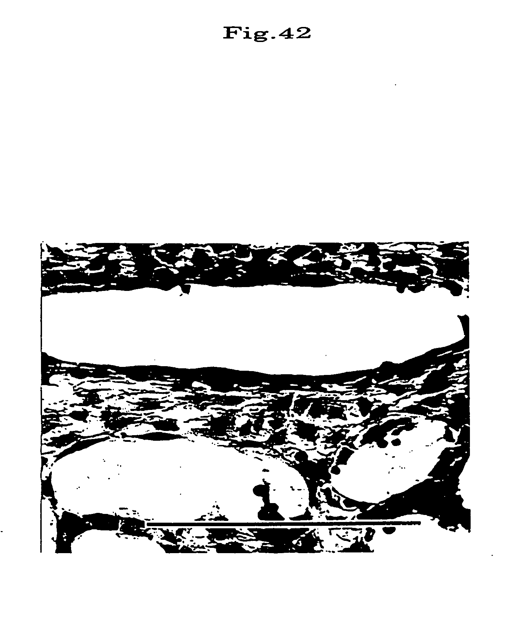

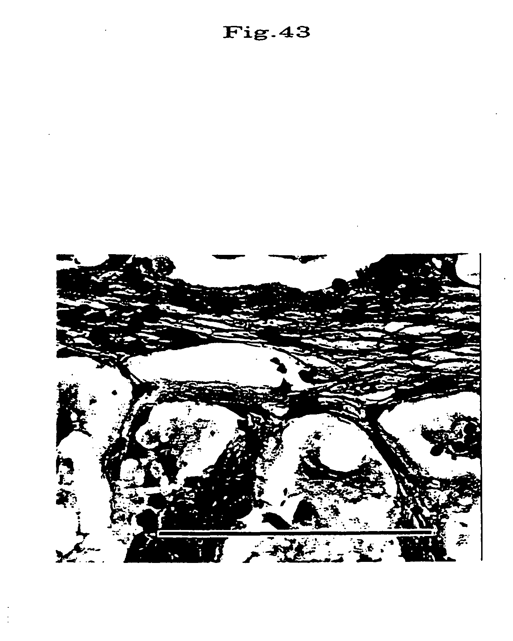

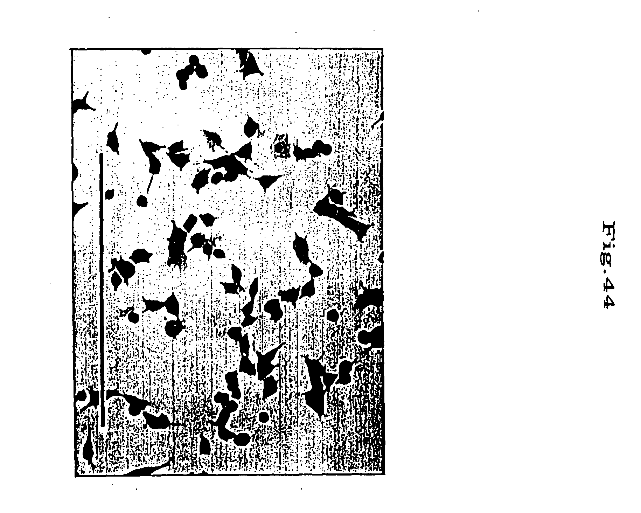

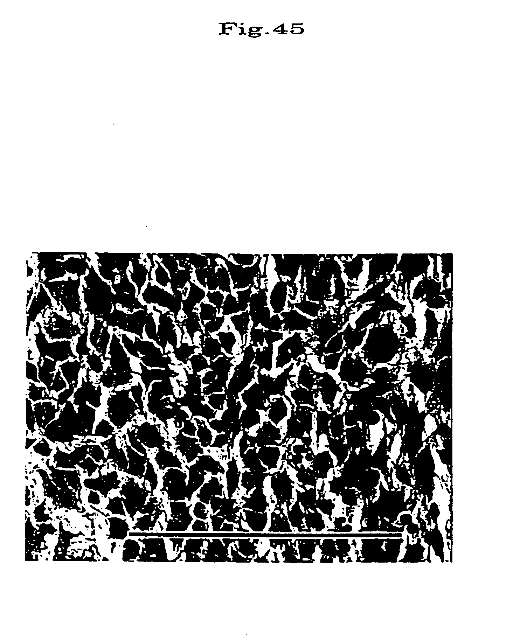

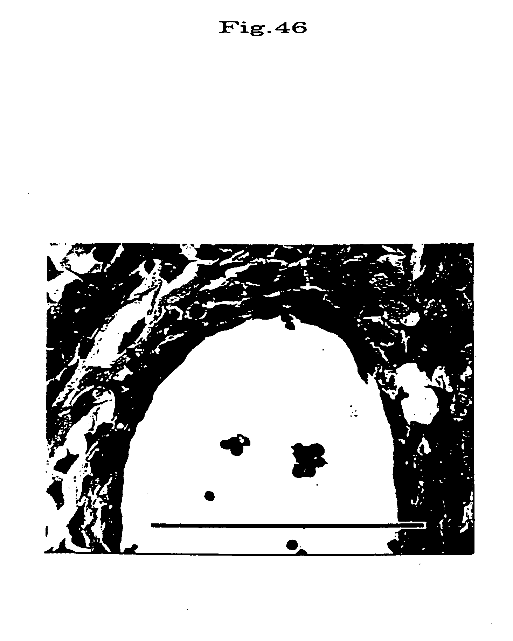

The slideglaess each having thereon the cell culture carrier of 5, 10 or 20 µm-thick frozen bovine placenta section that had been prepared in Example 1 were inserted into a culture tray. The PC-12 cells suspended in 20 ml of the culture medium were seeded in the tray to have a final cell density of 14.5 × 10⁴ cells/cm², and cultured in a humidified incubator containing 5 % CO₂ and 95 % air at 37°C. Four hours after the start of the culture, every slideglass was transferred from the tray into an individual 100-mmφ culture dish filled with 10 ml of the culture medium. After this, the cells were cultured for 2 days with changing the culture medium everyday. After thus cultured for 2 days, the slideglases were stained with hematoxylin-eosin, and observed with an optical microscope. No bovine placenta-derived cells were found on any of the 5, 10 and 20 µm-thick sections (see Figs. 41 to 43); and the cells cultured on these sections adhered and elongated more than those cultured on the slideglaess with no tissue section stuck thereto (see Fig. 44). In addition, it was found that, in the region corresponding to the cotyledon around the placental septa of the bovine placenta section, cobblestone-like cells principally adhered and elongated on the placental septa and squamous cells adhered and elongated along the edge of the placental septa (see Fig. 42); in the region which is rich in the connective tissue of the caruncle adjacent to the cotyledon in the bovine placenta section, cobblestone-like cells adhered and elongated (see Fig. 45); and in the region on the side of the lamina propria mucosae of the caruncle in the bovine placenta section, squamous cells adhered and elongated along the lumina of the endometrial glands and the blood vessels (see Fig. 46). The results suggest that the cultured cells recognized the microstructure of the tissue section on which they grew, and changed their morphology.

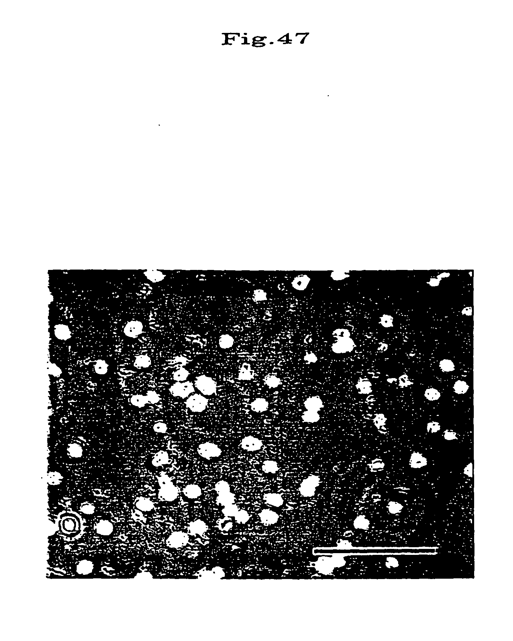

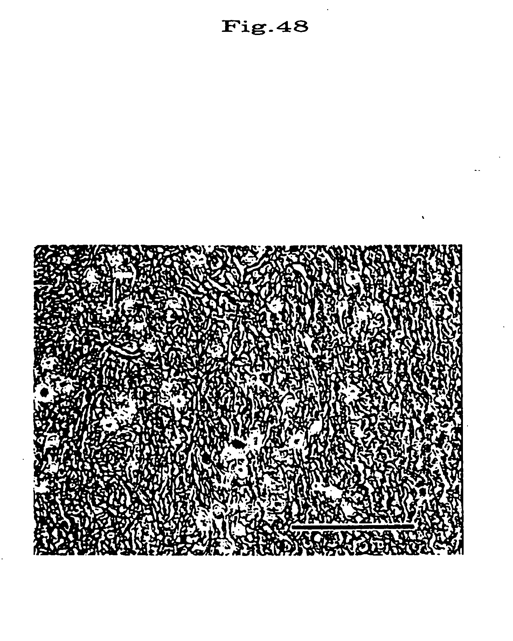

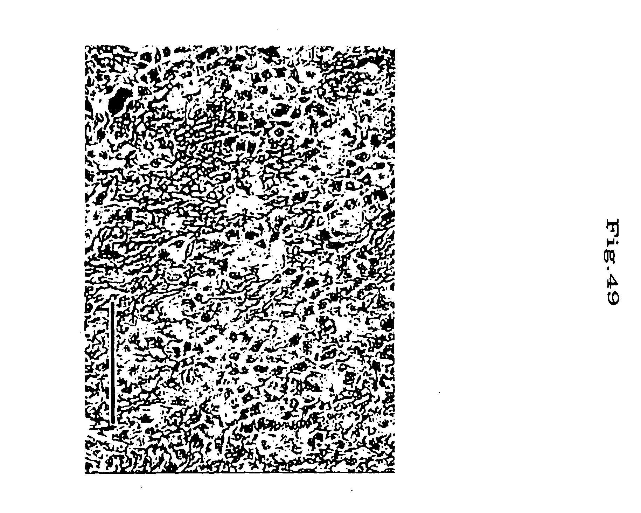

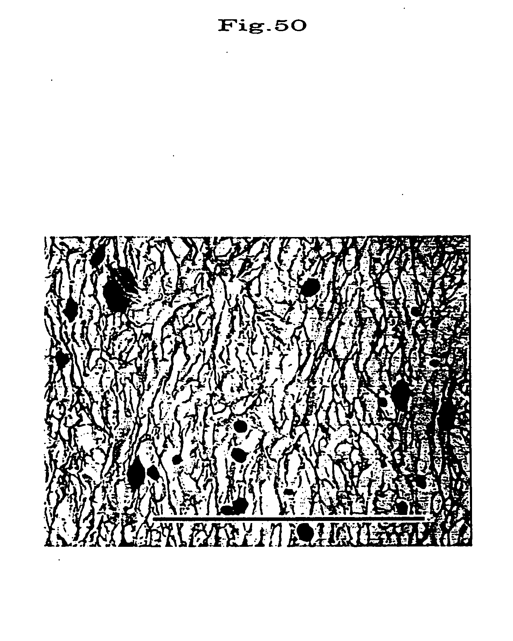

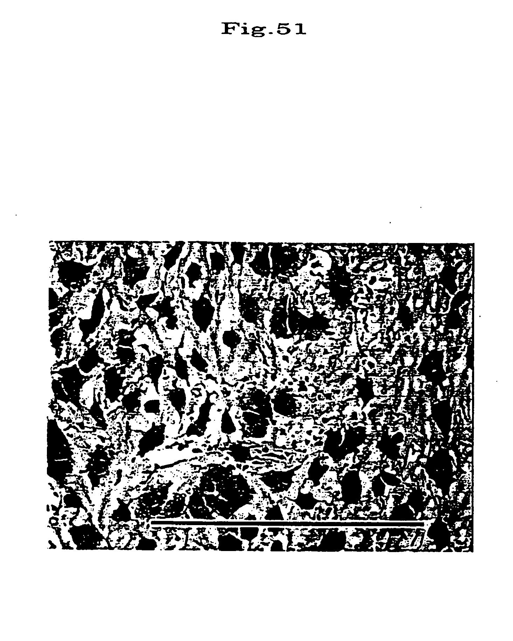

Next, the slideglases each having thereon the cell culture carrier of 5 µm- thick frozen bovine placenta section that had been prepared in Example 1, and the slideglases each having thereon the cell culture carrier of 5 µm-thick frozen bovine placenta section acellularized with 0.1 % SDS that had been prepared in Example 2 were inserted into a culture tray. The PC-12 cells suspended in 20 ml of the serum-free culture medium were seeded in the tray to have a final cell density of 4.6 × 10⁴ cells/cm², and cultured in a humidified incubator containing 5 % CO₂ and 95 % air at 37°C. Four hours after the start of the culture, every slideglass was transferred from the tray into an individual 100-mmϕ culture dish filled with 10 ml of the serum- free culture medium. After this, the cells were cultured for 5 days with changing the culture medium every other day. The cells were observed with a phase-contrast microscope for their morphology. The microscopic observation confirmed that, on day 5 of the culture, the cells on the slideglass with no tissue section stuck thereto almost all died (see Fig. 47), while some cells were still alive on the acellularized section, having adhered and spread like cobblestones (see Fig. 48), and almost all cells were alive on the section, having adhered and spread like cobblestones (see Fig. 49). On day 5 of the culture, the slideglases were stained with hematoxylin-eosin, and observed with an optical microscope. This confirmed that the cells on the section and on the acellularized section were alive, having adhered and spread like cobblestones. Specifically, on the section on which the cells were cultured, bovine placenta-derived cells were clearly seen; on the acellularized section on which the cells were cultured, a definite acellular tissue not having bovine placenta-derived cells was clearly seen (see Figs. 50, 51). These results suggest that the cell culture carrier of the bovine tissue section enables long-term living of the cells cultured in a serum-free medium, and that, when the cells are cultured in a serum-free medium, they can still maintain the section-derived cells and tissues even after cultured on the section for a long period of time.

Needless-to-say, the above-mentioned examples do not whatsoever restrict the scope of the invention. Plural tissue sections may be put on one slideglass with no limitation. Naturally, various types of animal tissues may be employable herein for forming the tissue sections, and, in addition, no limitation should be given to the animal cells to be cultured herein (apart from not being human embryonic cells), the condition of the sections, the type of the supports, the composition of the culture media, and the culture conditions. The invention of this application encompasses all modes and embodiments of carrying out it.

### INDUSTRIAL APPLICABILITY

The invention of this application provides a carrier for culture of animal cells, with which the culture environment is more similar to that in living tissue, or that is, it provides such a carrier for culture of animal cells which involves the intended, site and time-dependent information on living tissue. Culturing animal cells on the tissue-derived culture carrier of the invention makes it possible to analyze the properties and the characteristics of the cultured cells on the basis of the interaction between the cells and the site and time-dependent information on the tissue of the carrier, to analyze the function of genes on the basis of the interaction therebetween, to reconstruct a novel engineered tissue by utilizing the carrier tissue as a template, and to transplant the reconstructed tissue.

## Claims

1. A cell culture system comprising a cell culture vessel into which has been fitted a cell culture carrier which contains a tissue section which has been equilibrated with a culture medium, wherein
(i) the tissue section has previously been frozen and the tissue section has a thickness of from 1 to 50 µm, or
(ii) the tissue section has previously been embedded in paraffin and the tissue section has a thickness of from 0.5 to 50 µm, or
(iii) the tissue section has previously been embedded in resin and the tissue section has a thickness of from 0.5 to 50 µm,
and wherein animal cells have been seeded on the carrier to start the culture thereof, and wherein the said animal cells are animal cell suspensions, tissue explants, non-human blastocysts, or three-dimensionally reconstructed multicellular aggregates, but not human embryonic stem cells.

2. The cell culture system as claimed in claim 1, wherein the tissue section is adhered to a support or adhered to a support while stretched.

3. The cell culture system as claimed in claim 2, wherein the support is at least one selected from the group comprising glass, plastics, rubber, metal, natural or synthetic thread and/or material woven therefrom, and biodegradable materials.

4. The cell culture system as claimed in claim 2 or 3, wherein the support has previously been processed to promote adhesion of the section thereto or to promote adhesion of the section thereto under stretch.

5. The cell culture system as claimed in any one of claims 1to 4, wherein the tissue is prepared from a fresh tissue or a tissue previously fixed by the use of a fixing reagent.

6. The cell culture system as claimed in any one of claims 1 to 5, wherein the tissue is processed for acellularization before or after it has been sectioned.

7. The cell culture system as claimed in any one of claims 1 to 6, wherein the tissue section is treated with an antibody or a nucleic acid probe capable of binding a physiologically-active agent to introduce an exogenous physiologically-active agent into a specific site of the section, wherein the physiologically active agent is a protein, glycoprotein, lipoprotein, peptide, saccharide, lipid, liposaccharide, nucleic acid or a low molecular weight compound.

8. The cell culture system as claimed in any one of claims 1to 7, wherein the tissue section is biologically treated with an enzyme or chemically treated with acid, alkali or surfactant to change or modify the constituent components and/or the microstructure of the tissue section.

9. The cell culture system as claimed in any one of claims 1 to 8, wherein the tissue to be sectioned has previously been frozen, or embedded by freezing, or embedded in paraffin or resin.

10. The cell culture system as claimed in any one of claims 1 to 9, wherein the tissue section is derived from animals.

11. The cell culture system as claimed in any one of claims 1 to 10, wherein the tissue section is derived from mammals.

12. The cell culture system as claimed in any one of claims 1 to 11, wherein the tissue section is derived from a part or all of an unborn non-human animal living in its mother during the developmental stage.

13. The cell culture system as claimed in any one of claims 1to 11, wherein the tissue section is derived from a part or all of a born animal.

14. A method of cell culture, comprising fitting a cell culture carrier which contains a tissue section into a culture vessel, and culturing animal cells therein, wherein animal cell suspensions, tissue explants, non-human blastocysts, or three-dimensionally reconstructed multicellular aggregates, but not human embryonic stem cellsare seeded on the carrier to start the culture of the animal cells thereon.

15. The method as claimed in claim 14, wherein the tissue section is adhered to a support or adhered to a support while stretched.

16. The method as claimed in claim 15, wherein the support is at least one selected from the group comprising glass, plastics, rubber, metal, natural or synthetic thread and/or material woven therefrom, and biodegradable materials.

17. The method as claimed in claim 15, wherein the support has previously been processed to promote adhesion of the section thereto or to promote adhesion of the section thereto under stretch.

18. The method as claimed in any one of claims 14 to 17, wherein the tissue is prepared from a fresh tissue or a tissue previously fixed by the use of a fixing reagent.

19. The method as claimed in any one of claims 14 to 18, wherein the tissue is processed for acellularization before or after it has been sectioned.

20. The method as claimed in any one of claims 14 to 19, wherein the tissue section is treated with an antibody or a nucleic acid probe capable of binding a physiologically-active agent to introduce an exogenous physiologically-active agent into a specific site of the section, wherein the physiologically active agent is a protein, glycoprotein, lipoprotein, peptide, saccharide, lipid, liposaccharide, nucleic acid or a low molecular weight compound.

21. The method as claimed in any one of claims 14 to 20, wherein the tissue section is biologically treated with an enzyme or chemically treated with acid, alkali, or surfactant to change or modify the constituent components and/or the microstructure of the tissue section.

22. The method as claimed in any one of claims 14 to 21, wherein the tissue to be sectioned has previously been frozen, or embedded by freezing, or embedded in paraffin or resin.

23. The method as claimed in any one of claims 14 to 22, wherein the tissue section is derived from animals.

24. The method as claimed in any one of claims 14 to 23, wherein the tissue section is derived from mammals.

25. The method as claimed in any one of claims 14 to 24, wherein the tissue section is derived from a part or all of an unborn non-human animal living in its mother during the developmental stage.

26. The method as claimed in any one of claims 14 to 24, wherein the tissue section is derived from a part or all of a born animal.

27. The cell culture method as claimed in any one of claims 14 to 26 wherein the animal cells seeded on the carrier, adhere, proliferate, and the section or the section-derived tissue with the proliferated cells is subsequently peeled from the support of any of claims 15 to 17.

28. The cell culture method as claimed in claim 27, wherein the section or the section-derived tissue with the proliferated cells, after, having been peeled from the support, is further cultured to thereby form three-dimensional multicellular aggregates that contain the section or the section-derived tissue.

29. The cell culture method as claimed in claim 14, wherein the animal cells to be cultured are selected from the group consisting of primary culture cells, cells of established cell lines, non-human blastocysts and/or those cells with an exogenous gene introduced thereinto.

30. The cell culture method as claimed in claim 14, wherein the animal cells to be cultured are derived from any of undifferentiated stem cells, cells under differentiation, terminally differentiated cells and/or dedifferentiated cells, wherein the cells are not human embryonic cells.

31. The cell culture method as claimed in claim 30, wherein the undifferentiated stem cells are embryonic stem cells.

32. The cell culture method as claimed in any of claims 14 to 31, wherein the culture medium for culturing the animal cells therein is a serum-containing culture medium or a serum-free culture medium not containing serum.

## Patentansprüche

1. Zellkultursystem umfassend ein Zellkulturgefäß, in das ein Zellkulturträger eingepasst worden ist, der einen Gewebeschnitt enthält, der mit einem Kulturmedium equilibriert wurde, wobei
(i) der Gewebeschnitt zuvor eingefroren wurde und der Gewebeschnitt eine Dicke von 1 bis 50 µm aufweist, oder
(ii) der Gewebeschnitt zuvor in Paraffin eingebettet wurde und der Gewebeschnitt eine Dicke von 0,5 bis 50 µm aufweist, oder
(iii) der Gewebeschnitt zuvor in Harz eingebettet wurde und der Gewebeschnitt eine Dicke von 0,5 bis 50 µm aufweist,
und wobei tierische Zellen auf den Träger aufgeimpft wurden, um eine Kultur von diesen zu starten, und wobei die tierischen Zellen Suspensionen tierischer Zellen, Gewebeexplantate, nicht menschliche Blastozysten, oder dreidimensional rekonstruierte mehrzellige Aggregate, aber keine menschlichen embryonalen Stammzellen sind.

2. Zellkultursystem gemäß Anspruch 1, bei dem der Gewebeschnitt auf einer Auflage festgehalten ist oder in gedehnter Form auf einer Auflage festgehalten ist.

3. Zellkultursystem gemäß Anspruch 2, bei dem die Auflage mindestens eine Auflage ausgewählt aus der Gruppe, die Glas, Kunststoff, Gummi, Metall, natürliches oder synthetisches Garn und/oder daraus gewobenes Material, und biologisch abbaubare Materialien umfasst, ist.

4. Zellkultursystem gemäß Anspruch 2 oder 3, bei dem die Auflage zuvor bearbeitet worden ist, um die Haftung des Schnitts darauf zu fördern oder die Haftung des Schnitts darauf unter Dehnung zu fördern.

5. Zellkultursystem gemäß einem der Ansprüche 1 bis 4, bei dem das Gewebe aus einem frischen Gewebe oder einem Gewebe, das zuvor durch die Anwendung eines Fixiermittels fixiert wurde, präpariert ist.

6. Zellkultursystem gemäß einem der Ansprüche 1 bis 5, bei dem das Gewebe behandelt wird, um eine Lyse der Zellen zu bewirken (engl.: accellularization), bevor es geschnitten wird oder nachdem es geschnitten worden ist.

7. Zellkultursystem gemäß einem der Ansprüche 1 bis 6, bei dem der Gewebeschnitt mit einem Antikörper oder einer Nukleinsäuresonde mit der Fähigkeit, einen physiologisch aktiven Wirkstoff zu binden, behandelt ist, um einen exogenen physiologisch aktiven Wirkstoff in eine bestimmte Stelle des Schnittes einzuführen, wobei der physiologisch aktive Wirkstoff ein Protein, ein Glykoprotein, ein Lipoprotein, ein Peptid, ein Kohlehydrat, ein Lipid, ein Liposaccharid, eine Nukleinsäure oder eine niedermolekulare Verbindung ist.

8. Zellkultursystem gemäß einem der Ansprüche 1 bis 7, bei dem der Gewebeschnitt biologisch mit einem Enzym behandelt ist oder chemisch mit Säure, Alkali oder Netzmittel behandelt ist, um die konstituierenden Bestandteile und/oder die Mikrostruktur des Gewebeschnitts zu verändern oder zu modifizieren.

9. Zellkultursystem gemäß einem der Ansprüche 1 bis 8, bei dem das zu schneidende Gewebe zuvor eingefroren worden ist, oder durch Gefrieren eingebettet worden ist, oder in Paraffin oder Harz eingebettet worden ist.

10. Zellkultursystem gemäß einem der Ansprüche 1 bis 9, bei dem der Gewebeschnitt aus Tieren gewonnen ist.

11. Zellkultursystem gemäß einem der Ansprüche 1 bis 10, bei dem der Gewebeschnitt aus Säugetieren gewonnen ist.

12. Zellkultursystem gemäß einem der Ansprüche 1 bis 11, bei dem der Gewebeschnitt aus einem Teil oder der Gesamtheit eines ungeborenen, nicht menschlichen Tieres gewonnen ist, das während der Entwicklungsphase in seiner Mutter lebt.

13. Zellkultursystem gemäß einem der Ansprüche 1 bis 11, bei dem der Gewebeschnitt aus einem Teil oder der Gesamtheit eines geborenen Tieres gewonnen ist.

14. Verfahren zur Kultivierung von Zellen, umfassend das Einpassen eines Zellkulturträgers, der einen Gewebeschnitt enthält, in ein Zellkulturgefäß, und die Kultivierung von Tierzellen darin, wobei Suspensionen von Tierzellen, Gewebeexplantate, nicht menschliche Blastozysten oder dreidimensional rekonstruierte mehrzellige Aggregate, jedoch keine menschlichen embryonalen Stammzellen, auf den Träger aufgeimpft werden, um die Kultivierung der Tierzellen darauf zu starten.

15. Verfahren gemäß Anspruch 14, bei dem der Gewebeschnitt auf einer Auflage festgehalten wird oder in gedehnter Form auf einer Auflage festgehalten wird.

16. Verfahren gemäß Anspruch 15, bei dem die Auflage mindestens eine Auflage ausgewählt aus der Gruppe, die Glas, Kunststoff, Gummi, Metall, natürliches oder synthetisches Garn und/oder daraus gewobenes Material, und biologisch abbaubare Materialien umfasst, ist.

17. Verfahren gemäß Anspruch 15, bei dem die Auflage zuvor bearbeitet worden ist, um die Haftung des Schnitts darauf zu fördern oder die Haftung des Schnitts darauf unter Dehnung zu fördern.

18. Verfahren gemäß einem der Ansprüche 14 bis 17, bei dem das Gewebe aus einem frischen Gewebe oder einem Gewebe, das zuvor durch die Anwendung eines Fixiermittels fixiert wurde, präpariert wird.

19. Verfahren gemäß einem der Ansprüche 14 bis 18, bei dem das Gewebe behandelt wird, um eine Lyse der Zellen zu bewirken, (engl.: acellularization) bevor es geschnitten wird oder nachdem es geschnitten worden ist.

20. Verfahren gemäß einem der Ansprüche 14 bis 19, bei dem der Gewebeschnitt mit einem Antikörper oder einer Nukleinsäuresonde mit der Fähigkeit, einen physiologisch aktiven Wirkstoff zu binden, behandelt wird, um einen exogenen physiologisch aktiven Wirkstoff in eine bestimmte Stelle des Schnittes einzuführen, wobei der physiologisch aktive Wirkstoff ein Protein, ein Glykoprotein, ein Lipoprotein, ein Peptid, ein Kohlehydrat, ein Lipid, ein Liposaccharid, eine Nukleinsäure oder eine niedermolekulare Verbindung ist.

21. Verfahren gemäß einem der Ansprüche 14 bis 20, bei dem der Gewebeschnitt biologisch mit einem Enzym behandelt wird oder chemisch mit Säure, Alkali oder Netzmittel behandelt wird, um die konstituierenden Bestandteile und/oder die Mikrostruktur des Gewebeschnitts zu verändern oder zu modifizieren.

22. Verfahren gemäß einem der Ansprüche 14 bis 21, bei dem das zu schneidende Gewebe zuvor eingefroren worden ist, oder durch Gefrieren eingebettet worden ist, oder in Paraffin oder Harz eingebettet worden ist.

23. Verfahren gemäß einem der Ansprüche 14 bis 22, bei dem der Gewebeschnitt aus Tieren gewonnen wird.

24. Verfahren gemäß einem der Ansprüche 14 bis 23, bei dem der Gewebeschnitt aus Säugetieren gewonnen wird.

25. Verfahren gemäß einem der Ansprüche 14 bis 24, bei dem der Gewebeschnitt aus einem Teil oder der Gesamtheit eines ungeborenen, nicht menschlichen Tieres gewonnen wird, das während der Entwicklungsphase in seiner Mutter lebt.

26. Verfahren gemäß einem der Ansprüche 14 bis 24, bei dem der Gewebeschnitt aus einem Teil oder der Gesamtheit eines geborenen Tieres gewonnen wird.

27. Verfahren zur Kultivierung von Zellen gemäß einem der Ansprüche 14 bis 26, bei dem die auf den Träger aufgeimpften Tierzellen anhaften und sich vermehren und der Schnitt oder das aus dem Schnitt gewonnene Gewebe mit den vermehrten Zellen anschließend abgeschält wird von dem Träger gemäß einem der Ansprüche 15 bis 17.

28. Verfahren zur Kultivierung von Zellen gemäß Anspruch 27, bei dem der Schnitt oder das aus dem Schnitt gewonnene Gewebe mit den vermehrten Zellen nach dem Abschälen vom Träger weiter kultiviert wird, um **dadurch** dreidimensionale mehrzellige Aggregate zu bilden, die den Schnitt oder das aus dem Schnitt gewonnene Gewebe enthalten.

29. Verfahren zur Kultivierung von Zellen gemäß Anspruch 14, bei dem die zu kultivierenden Tierzellen ausgewählt sind aus der Gruppe bestehend aus Primärkulturzellen, Zellen aus etablierten Zelllinien, nicht menschlichen Blastozysten und/oder derartigen Zellen, in die ein exogenes Gen eingeführt wurde.

30. Verfahren zur Kultivierung von Zellen gemäß Anspruch 14, bei dem die zu kultivierenden Tierzellen sich ableiten von irgendwelchen undifferenzierten Stammzellen, sich gerade differenzierenden Zellen, terminal differenzierten Zellen und/oder rückdifferenzierten Zellen, wobei die Zellen keine menschlichen embryonalen Zellen sind.

31. Verfahren zur Kultivierung von Zellen gemäß Anspruch 30, bei dem die undifferenzierten Stammzellen embryonale Stammzellen sind.

32. Verfahren zur Kultivierung von Zellen gemäß einem der Ansprüche 14 bis 31, bei dem das Kulturmedium, in dem die Tierzellen gezüchtet werden, ein Serum enthaltendes Kulturmedium oder ein serumfreies Kulturmedium, das kein Serum enthält, ist.

## Revendications

1. Système de culture de cellules comprenant un récipient de culture de cellules
dans lequel a été ajusté un support de culture de cellules qui contient une coupe d'un tissu qui a été équilibrée avec un milieu de culture, dans lequel :
(i) la coupe de tissu a été congelée au préalable et la coupe de tissu a une épaisseur comprise entre 1 et 50 µm, ou
(ii) la coupe de tissu a été incluse au préalable dans de la paraffine et la coupe de tissu a une épaisseur comprise entre 0,5 et 50 µm, ou
(iii) la coupe de tissu a été incluse au préalable dans une résine et la coupe de tissu a une épaisseur comprise entre 0,5 et 50 µm,
dans lequel des cellules animales ont été ensemencées sur le support pour en démarrer la culture, et dans lequel lesdites cellules animales sont des suspensions de cellules animales, des explants de tissus, des blastocystes non-humains ou des agrégats multicellulaires reconstruits de manière tridimensionnelle, mais ne sont pas des cellules souches embryonnaires humaines.

2. Système de culture de cellules selon la revendication 1, dans lequel la coupe de tissu est collée à un support ou collée à un support sous étirement.

3. Système de culture de cellules selon la revendication 2, dans lequel le support est au moins un support choisi parmi le groupe comprenant le verre, le plastique, le caoutchouc, le métal, un fil naturel ou synthétique et/ou un matériau tissé avec celui-ci et des matériaux biodégradables.

4. Système de culture de cellules selon la revendication 2 ou 3, dans lequel le support a été traité au préalable afin de faciliter l'adhésion de la coupe à celui-ci ou l'adhésion de la coupe sous étirement à celui-ci.

5. Système de culture de cellules selon l'une quelconque des revendications 1 à 4, dans lequel le tissu est préparé à partir d'un tissu frais ou d'un tissu fixé au préalable à l'aide d'un fixateur.

6. Système de culture de cellules selon l'une quelconque des revendications 1 à 5, dans lequel le tissu est traité pour éliminer les cellules avant ou après la préparation des coupes.

7. Système de culture de cellules selon l'une quelconque des revendications 1 à 6, dans lequel la coupe de tissu est traitée avec un anticorps ou une sonde d'acide nucléique susceptible de se lier à un agent possédant une activité physiologique afin d'introduire un agent exogène possédant une activité physiologique au niveau d'un site spécifique de la coupe, l'agent possédant une activité physiologique étant une protéine, une glycoprotéine, une lipoprotéine, un peptide, un saccharide, un lipide, un saccharolipide, un acide nucléique ou un composé de faible poids moléculaire.

8. Système de culture de cellules selon l'une quelconque des revendications 1 à 7, dans lequel la coupe de tissu est soumise à un traitement biologique avec une enzyme ou à un traitement chimique avec un acide, un alcali ou un agent tensioactif afin de changer ou de modifier les composants constitutifs et/ou la microstructure de la coupe de tissu.

9. Système de culture de cellules selon l'une quelconque des revendications 1 à 8, dans lequel le tissu à couper a, au préalable, été congelé, ou inclus par congélation, ou inclus dans de la paraffine ou dans une résine.

10. Système de culture de cellules selon l'une quelconque des revendications 1 à 9, dans lequel la coupe de tissu provient d'animaux.

11. Système de culture de cellules selon l'une quelconque des revendications 1 à 10, dans lequel la coupe de tissu provient de mammifères.

12. Système de culture de cellules selon l'une quelconque des revendications 1 à 11, dans lequel la coupe de tissu provient d'une partie ou de la totalité d'un animal non humain non-né vivant dans sa mère pendant le stade de développement.

13. Système de culture de cellules selon l'une quelconque des revendications 1 à 11, dans lequel la coupe de tissu provient d'une partie ou de la totalité d'un animal né.

14. Procédé de culture de cellules, comprenant l'ajustement d'un support de culture de cellules qui porte une coupe de tissu dans un récipient de culture et la culture de cellules animales dans celui-ci, dans lequel des suspensions de cellules animales, des explants de tissus, des blastocytes non-humains ou des agrégats multicellulaires reconstruits en trois dimensions, à l'exclusion des cellules souches embryonnaires humaines, sont ensemencés sur le support afin de démarrer la culture de cellules animales sur celui-ci.

15. Procédé selon la revendication 14, dans lequel la coupe de tissu est collée à un support ou collée à un support sous étirement.

16. Procédé selon la revendication 15, dans lequel le support est au moins un support choisi parmi le groupe comprenant le verre, le plastique, le caoutchouc, le métal, un fil naturel ou synthétique et/ou un matériau tissé avec celui-ci et des matériaux biodégradables.

17. Procédé selon la revendication 15, dans lequel le support a été traité au préalable afin de faciliter l'adhésion à celui-ci de la coupe ou pour faciliter l'adhésion à celui-ci de la coupe sous étirement.

18. Procédé selon l'une quelconque des revendications 14 à 17, dans lequel le tissu est préparé à partir d'un tissu frais ou d'un tissu fixé au préalable à l'aide d'un fixateur.

19. Procédé selon l'une quelconque des revendications 14 à 18, dans lequel le tissu est traité pour éliminer les cellules avant ou après la préparation des coupes.

20. Procédé selon l'une quelconque des revendications 14 à 19, dans lequel la coupe de tissu est traitée avec un anticorps ou une sonde d'acide nucléique susceptible de se lier à un agent possédant une activité physiologique afin d'introduire un agent exogène possédant une activité physiologique au niveau d'un site spécifique de la coupe, l'agent possédant une activité physiologique étant une protéine, une glycoprotéine, une lipoprotéine, un peptide, un saccharide, un lipide, un saccharolipide, un acide nucléique ou un composé de faible poids moléculaire.

21. Procédé selon l'une quelconque des revendications 14 à 20, dans lequel la coupe de tissu est soumise à un traitement biologique avec une enzyme ou à un traitement chimique avec un acide, un alcali ou un agent tensioactif afin de changer ou de modifier les composants constitutifs et/ou la microstructure de la coupe de tissu.

22. Procédé selon l'une quelconque des revendications 14 à 21, dans lequel le tissu à couper a, au préalable, été congelé, ou inclus par congélation, ou inclus dans de la paraffine ou dans une résine.

23. Procédé selon l'une quelconque des revendications 14 à 22, dans lequel la coupe de tissu provient d'un animal.

24. Procédé selon l'une quelconque des revendications 14 à 23, dans lequel la coupe de tissu provient d'un mammifère.

25. Procédé selon l'une quelconque des revendications 14 à 24, dans lequel la coupe de tissu provient d'une partie ou de la totalité d'un animal non humain non-né vivant dans sa mère pendant le stade de développement.

26. Procédé selon l'une quelconque des revendications 14 à 24, dans lequel la coupe de tissu provient d'une partie ou de la totalité d'un animal né.

27. Procédé de culture de cellules selon l'une quelconque des revendications 14 à 26, dans lequel les cellules animales ensemencées sur le support adhèrent, prolifèrent, et la coupe ou le tissu dérivé de la coupe contenant des cellules qui ont proliféré est ensuite détaché du support selon l'une quelconque des revendications 15 à 17.

28. Procédé de culture de cellules selon la revendication 27, dans lequel la coupe ou le tissu dérivé de la coupe contenant des cellules qui ont proliféré, après avoir été détaché du support, est encore cultivé de manière à former des agrégats multicellulaires tridimensionnels qui contiennent la coupe ou le tissu dérivé de la coupe.

29. Procédé de culture de cellules selon la revendication 14, dans lequel les cellules animales à cultiver sont sélectionnées dans le groupe constitué des cellules en culture primaire, des cellules de lignées cellulaires établies, des blastocystes non humains et/ou des cellules dans lesquelles a été introduit un gène exogène.

30. Procédé de culture de cellules selon la revendication 14, dans lequel les cellules animales à cultiver proviennent indifféremment de cellules souches non différenciées, de cellules en cours de différenciation, de cellules compléments différenciées et/ou de cellules dédifférenciées, lesquelles cellules n'étant pas des cellules embryonnaires humaines.

31. Procédé de culture de cellules selon la revendication 30, dans lequel les cellules souches non différenciées sont des cellules souches embryonnaires.

32. Procédé de culture de cellules selon l'une quelconque des revendications 14 à 31, dans lequel le milieu de culture dans lequel sont cultivées les cellules animales est un milieu de culture contenant du sérum ou un milieu de culture sans sérum ne contenant pas de sérum.
